(19) Europäisches Patentamt
European Patent Office
Office européen des brevets

(11) EP 4 201 508 A1

# (12) EUROPEAN PATENT APPLICATION

(43) Date of publication:
**28.06.2023 Bulletin 2023/26**

(21) Application number: **21216683.9**

(22) Date of filing: **21.12.2021**

(51) International Patent Classification (IPC):
***B01D 67/00*** ^(2006.01) ***A61M 1/34*** ^(2006.01)
***B01D 71/44*** ^(2006.01) ***B01D 71/68*** ^(2006.01)

(52) Cooperative Patent Classification (CPC):
**B01D 67/0093; B01D 67/0088; B01D 71/441; B01D 71/68;** A61M 1/1621; A61M 1/34; A61M 1/3673; B01D 2313/68; B01D 2323/2187

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Gambro Lundia AB**
**220 10 Lund (SE)**

(72) Inventors:
- **SWARTJES, Jan J.T.M.**
  **72072 Tübingen (DE)**
- **VOTTELER, Stefanie**
  **72768 Reutlingen (DE)**
- **STORR, Markus**
  **70794 Filderstadt (DE)**
- **HORNUNG, Markus**
  **72147 Nehren (DE)**

(74) Representative: **Hornung, Veronika Margot**
**Gambro Dialysatoren GmbH**
**Holger-Crafoord-Strasse 26**
**72379 Hechingen (DE)**

## (54) MEMBRANE COATED WITH POLYDOPAMINE AND CHONDROITIN AND PROCESS FOR PRODUCING SAME

(57) The present disclosure relates to a membrane which is coated with polydopamine and chondroitin comprising a blend of i) a polysulfone, polyethersulfone or polyarylethersulfone and ii) polyvinylpyrrolidone, characterized in that the membrane is coated with polydopamine and chondroitin. Optionally the coating further comprises heparin. The disclosure further relates to a process for producing the coated membrane, a method of increasing the selectivity of a membrane, and to a filtration/diffusion device comprising the membrane which can be used, for example, in hemodialysis applications.

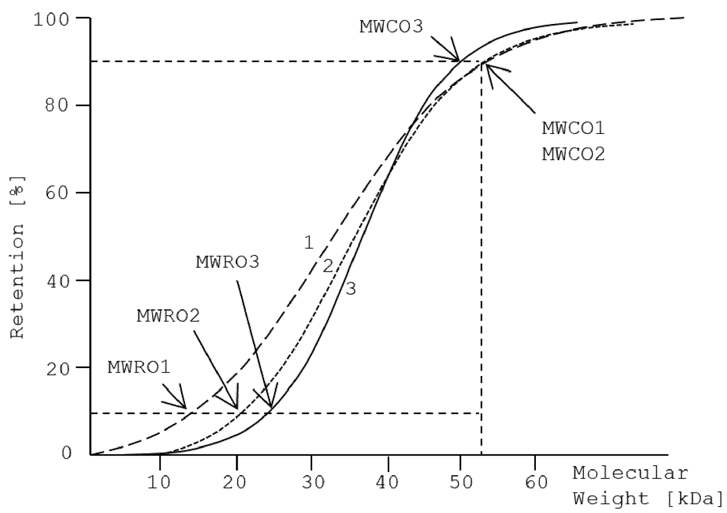


Figure 1



Processed by Luminess, 75001 PARIS (FR)

## Description

### Technical Field

The disclosure relates to a membrane which is coated with polydopamine and chondroitin comprising a blend of i) a polysulfone, polyethersulfone or polyarylethersulfone and ii) polyvinylpyrrolidone, characterized in that the membrane is coated with polydopamine and chondroitin. Optionally the coating further comprises heparin. The disclosure further relates to a process for producing the coated membrane, a method of increasing the selectivity of a membrane, and to a filtration/diffusion device comprising the membrane which can be used, for example, in hemodialysis applications.

### Description of the Related Art

Patients with end stage renal disease in hemodialysis have an increased mortality risk when compared to the general population. This indicates that the currently available replacement therapies, even though relying on advanced membrane and dialyzer technologies as well as highly effective and sophisticated machines, should still be improved in a way that the clinical outcome for patients and their quality of life during therapy is getting better. One of the still manifold unmet needs that has been accompanying the development of new membranes and dialyzers over decades is the enhanced removal of uremic toxins over a broad range and including uremic toxins of a higher molecular weight, while essentially retaining proteins such as albumin and other such higher molecular weight compounds that are deemed to be critical. In other words, synthetic membranes which are state of the art today are still less selective than the glomerular membrane of the kidney, and so higher molecular weight proteins are not cleared from the patients' blood appropriately. In addition, hemodialysis is performed generally thrice weekly for approximately 4 hours, while the healthy kidney operates continuously (Boschetti-de-Fierro et al., Scientific Reports (2015) 5: 18448), which adds to the issues around the accumulation of uremic toxins in the body. Another persistent need in hemodialysis is the hemocompatibility of the materials used in the treatment, specifically the hemocompatibility of membranes that are in direct blood contact, including their thrombogenicity that requires using systemic heparin in most therapies.

Accordingly, membrane innovation has recently been focused on the enhanced removal of larger molecular weight uremic toxins and increased membrane permeability, and the improvement of hemocompatibility and antithrombogenicity of the membranes. The progressive shift towards increased membrane permeability and concomitant increased selectivity is driven by said gap between synthetic membranes and the natural kidney which combines both high permeability and high selectivity.

As mentioned above, typical hallmarks of selectivity of a membrane specifically for use in hemodialysis are the sieving coefficients for albumin (~66.4kDa) on the one hand and the sieving coefficients for large middle molecules (>25-58kDa) on the other hand (Rosner et al., Classification of Uremic Toxins and Their Role in Kidney Failure: CJASN 16, 1-8 (2021)). Such large middle molecules include, for example, YKL-40 (~40kDa), AGEs, lambda-FLC, CX3CL1, CXCL12, or IL-2. However, selectivity does expressly encompass the highly efficient removal of also small middle molecules (0.5-15kDa) such as β2-microglubulin, and medium middle molecules (>15-25kDa), such as TNF, IL-10, IL-6, kappa-FLC, myoglobin, FGF-2, complement factor D and others. The sieving coefficients for said molecules are reflected, for example, in the sieving curve and as further represented by the MWCO and MWRO values of a membrane which provide information on the onset of retention of molecules, i.e., where the sieving coefficient is 0.9, and the cut-off of a membrane, i.e., where the sieving coefficient is 0.1. Together, MWRO and MWCO define the steepness of the sieving curve and thus provide for an excellent measure of the selectivity of a membrane. For example, shifting the retention onset to higher molecular weights while keeping the cut-off stable or reducing it results in increased selectivity, which is apparent from an increased steepness of the sieving curve (**FIG. 1**). Keeping the MWRO of a membrane stable while reducing the MWCO thereof could be another way to improve the selectivity of a membrane.

Hemodialyzers comprising such selective membranes should have a low albumin loss over a typical dialysis treatment of about 4 hours (or simulated use correlated with *in vivo* use), i.e. the albumin loss should remain below about 7 g/4h, and preferably should remain below about 4g/4h. At the same time, the hemodialyzers should provide for a good clearance for large middle molecules, including, for example, YKL-40, i.e., they should have a YKL-40 clearance of at least 20 mL/min and higher. They should, in addition, efficiently remove β-2 microglobulin (β2m) with a clearance of more than 80 mL/min.

β-2 microglobulin is a well-known marker molecule in hemodialysis. It is a component of MHC class I molecules with a molecular weight of 11 kDa that in patients on long-term hemodialysis can aggregate into amyloid fibers that deposit in joint spaces, a disease, known as dialysis-related amyloidosis. YKL-40 (or chitinase-3-like protein) has recently emerged as a relevant and functional marker protein in hemodialysis (Lorenz et al., Kidney International (2018) 93: 221-230), where it has been linked to chronic inflammatory response in chronic kidney disease and HD patients. Its presence in the serum of the patients makes it accessible and traceable.

Recently, a significant step towards membranes and hemodialyzers with such improved performance was

made by the introduction of so-called MCO membranes and dialyzers, which are characterized by membranes having a high molecular weight retention onset (MWRO), thereby allowing also larger molecular weight toxins to pass the membrane to a significant extent, combined with a reasonably low molecular weight cut off (MWCO), that implicates that still proteins and other compounds that have a molecular weight of albumin and higher are essentially retained (WO 2015/118046A1 and WO 2015/118046A1). In other words, the design of the membrane allows for a significantly improved selectivity, which is a distinguishing feature over other prior art membranes such as the so-called "high cut-off" (HCO) membranes or membranes that have been referred to, in the past, as "protein-leaking" (PL) membranes. While HCO membranes are also able to efficiently remove the said higher molecular weight toxins, their increased albumin loss recommends their use only in acute situations that are limited in time and strictly controlled as regards albumin loss and replacement thereof. Protein-leaking membranes, on the other hand, generally have a cut-off that provides for a limited loss of albumin and other higher molecular weight compounds beyond albumin, but the retention onset is generally low, resulting in a flat sieving curve that signifies a low selectivity. In contrast, MCO membranes when combined with a careful physical design of the hollow fibers, of the membrane bundles and the overall dialyzer, lead to a performance which provides for an unprecedented clearance of relevant higher molecular weight toxins in hemodialysis, while albumin loss can be kept within narrow limits (Kirsch et al., Nephrology Dialysis Transplantation (2017) 32: 165). They thus provide for an excellent selectivity.

The significant contribution of MCO membranes to the final superior dialyzer performance is achieved by a careful control of all relevant parameters of the manufacturing process as described in WO 2015/118046A1 and WO 2015/118046A1. Such highly controlled manufacturing process is not easily reproducible throughout the industry, and further improving the selectivity of MCO membranes by further improving the process is difficult or at least very expensive. However, as mentioned above, it would be desirable to further improve this and also other, more common membranes towards even higher selectivity by easily accessible methods that allow the production of highly selective hemodialysis membranes without excessive costs that add to the price of the final product.

Other membranes, such as standard hemodialysis membranes and dialyzers, including, for example, high-flux membranes that are today widely used in hemodialysis (HD) could also benefit from methods to improve their selectivity in a simple, cost-effective, and safe way to provide access to improved dialyzers to more patients that may otherwise not be able to benefit from potentially more expensive high-end dialyzers as discussed above or potentially even more expensive therapies such as HDF. Clearly, any such methods for improving the selectivity of available membranes and dialyzers must guarantee the biocompatibility of the membrane and the dialyzer and its general safety for the patient.

The modification of membranes, including hemodialysis membranes, by immobilizing biologically active or inactive compounds, e.g., for improving their biocompatibility, antithrombogenicity, or performance, or for adding certain specific or unspecific properties, e.g., for capturing certain compounds such as, for example, hydrophilic or hydrophobic molecules, or for specifically binding certain molecules with the help of immobilized antibodies against such molecules have been described in the prior art.

For example, US 2003/0021826 A1 proposes binding an anticoagulation agent in a stable manner to the surface of semipermeable support membranes essentially comprised of a copolymer of acrylonitrile and at least one anionic or anionizable monomer. The anticoagulation agent can exert its anticoagulating activity without being leached out into the blood or plasma during treatment by extracorporeal circulation and to reduce the quantity of anticoagulation agent used systemically in the patient during an extracorporeal blood treatment session. The surface of the semipermeable support membrane intended to be brought into contact with the blood or plasma is coated in succession with a cationic polymer carrying cationic groups which can form an ionic bond with anionic or anionizable groups of polyacrylonitrile, for example, polyethyleneimine (PEI), and an anticoagulation agent carrying anionic groups capable of forming an ionic bond with cationic groups of said cationic polymer (for example, heparin). Membranes based on this technology are today used in hemodialysis, for example in the Evodial or Oxiris dialyzer (both from Baxter Int., Inc.).

Typically, the hemodialysis treatment requires the dosage of heparin to the patient to prevent blood clotting on the artificial membrane surface. Heparin activates antithrombin, which inhibits factor X as well as thrombin. Due to this influence on the coagulation cascade, the formation of a thrombus in the human blood is disturbed. Furthermore, heparin significantly reduces platelet deposition. In clinical treatments, the dosage of heparin is not generally individualized. Therefore, it is administered in larger amounts and results in long waiting times until the heparin is degraded in the patients' blood after each treatment procedure. The immobilization of heparin on the membrane surface is a promising approach to prevent the heparin administration and resulting long-term damages.

Another compound which has been widely used already for modifying membranes alone or in combination with other materials is polydopamine. It has been used for the modification in various membrane processes and for various purposes.

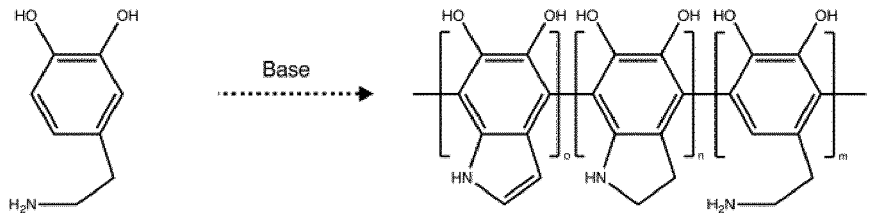


Dopamine Polydopamine

Polydopamine is obtained from dopamine, generally in an alkaline environment or through oxidation and adheres to various surfaces. The variety of reactive groups provides for many possibilities for functionalizing surfaces for practical applications in material sciences, biomedicine, surface engineering and catalysis (Liebscher, Chemistry of Polydopamine - Scope, Variation, and Limitation. Eur. J. Org. Chem., 2019 (2019): 4976-4994).

For example, WO 2012/047282 A2 describes methods of enhancing the anti-fouling properties of TFC-FO membranes by coating them with a mixture of dopamine and anti-fouling polymers. The addition of dopamine to the reaction mixture enables the formation of a more stable coating.

US 2010/0059433 A1 discloses depositing a coating material on a membrane used for purifying contaminated water to reduce or prevent biofilm formation on the membrane surface by adding a dopamine coating material to a liquid solvent to form a solution mixture, adjusting a pH of the solution mixture to 8, 9, or 10 and dissolving the dopamine coating material in the liquid solvent. The solution mixture is then placed into contact with the membrane surfaces to form a dopamine coating on the surface to reduce biofilm formation.

WO 2013/016574 A1 describes a thin film composite osmosis membrane having two layers, said layers comprising a composite layer comprising a backing layer embedded in a porous polymer-based support, and a rejection layer disposed on the composite layer, wherein the porous polymer-based support is based on polysulfone, polyethersulfone, sulfonated polysulfone or sulfonated polyethersulfone and mixtures thereof, and wherein the composite layer may be coated with polydopamine. The membrane of the invention exhibits high water flux values for FO processes.

US 2016/0101390 A1 discloses a method for modifying polymeric membranes to mitigate biofouling. More particularly, dopamine powder is dissolved in a buffer solution, and the membrane surface is exposed to the solution, resulting in the formation of a polydopamine thin film on the membrane. The surface of the polydopamine-modified membrane is then exposed to $AgNO_3$ solutions, resulting in the formation of silver nanoparticles (AgNPs) on the membrane surface.

EP 2695668 A1 describes the manufacturing of thermoresponsive filtration membranes, such as microfiltration membranes, wherein the filtration membrane is coated with a dopamine solution, followed by polymerizing to form polydopamine and immersing the coated membrane in a solution containing poly(N-isopropylacrylamide). The polydopamine coated membrane is less prone to blockage of the membrane pores by compounds present in the solution to be filtered and is thus less prone to fouling.

Gao et al., Journal of Membrane Science 452 (2014) : 390-399 describe the production of a bio-based poly (lactic acid) (PLA) membrane with asymmetric porous structure for hemodialysis to which heparin was immobilized surface via dopamine. The surface heparinization mediated by dopamine improved the hemocompatibility of the PLA membrane, suppressed the adhesion of platelets and reduced hemolysis.

Li et al., Desalination 344 (2014): 422-430 describe the modification of polyethersulfone (PES) membranes with different MWCO (molecular weight cut off) by polydopamine (PD) coating and PD-graft-poly(ethylene glycol) (PD-g-PEG) modification, resulting in less flux reduction in filtration and lower adsorptive amount of BSA in isothermal adsorption tests.

Polydopamine is also described in WO 2017/015291A1, where it is used for conditioning of membranes for use in water purification for extending their lifetime.

Chondroitin is another compound that has been used as a component for modifying structures or materials. The expression "chondroitin" as used herein refers to chondroitin sulfate (CS). CS is an anionic linear polysaccharide, which is synthesized as part of proteoglycan (PG) molecules in vertebrates and invertebrates. CS with different disaccharides and overall carbohydrate backbones may be biosynthesized possessing various numbers and positions of sulfate groups. Consequently, CS is a largely heterogeneous molecule formed of alternate and variously sulfated disaccharides of GlcA and GalNAc linked by β(1→3) bonds. Moreover, the different disaccharides are linked to each other by β(1→4) bonds. CS-A and CS-C are the predominant CS variants and are constituted by disaccharides sulfated in position C4 or C6 of the GalNAc residue, respectively, and minor percentages of the monosulfated disaccharide on C2 of GlcA are possible. Besides the main presence of these two kinds of disaccharide units monosulfated in position C4 or C6 of GalNAc, disaccharides with a different number and position of sulfate groups can be present, in various percentages, within the carbohydrate chains (Volpi, molecules (2019) 24: 1447). CS provides for various biological functions at molecular, cellular and organ level, e.g., in cell adhesion, division and differentiation, and in neural network formation. CS is present in all mammalian connective tissues, especially in the cartilage, skin, blood vessels, ligaments and tendons with different structure and level of sulfonation.

In Ning et al., Progress in Polymer Science (2018) 81: 144-162, polypyrrole (PPy) nanostructures for use as regenerative biomaterials in tissue regeneration were doped with, for example, chondroitin sulfate (CS).

WO 2017/030502 A1 describes biomimetic membranes for use in nanofiltration. The membranes have deposited thereon a first mixture comprising a first polyelectrolyte and a transmembrane protein such as aquaporin, and a second mixture comprising a second polyelectrolyte and a cross-linking agent, wherein the second polyelectrolyte has a charge opposite to the charge on the first polyelectrolyte. Chondroitin sulfate is mentioned as an anionic polyelectrolyte which can be used for producing such biomimetic membranes.

Han et al., ACS Applied Materials & Interfaces (2018) 10: 28015-28026 describe polydopamine-chondroitin sulfate-polyacrylamide (PDA-CS-PAM) hydrogels with tissue adhesiveness and superior mechanical properties for growth-factor-free cartilage regeneration.

In Yuan et al., Desalination (2008) 234: 166, chondroitin sulfate (CS) was used to improve the hydrophilicity of porous chitosan (CH) membranes fabricated by freeze-gelation. CS was cross-linked to CH by ionic interaction and covalent cross-linking using EDC/NHS coupling reagents. The pore structures, mechanical properties, and surface hydrophilicity of the porous chondroitin sulfate-modified chitosan (CH/CS) membranes could be altered by varying the weight ratio of CS to CH. The CH/CS membranes, due to their higher mechanical strength, hydrophilicity, and better cell compatibility, are proposed as biomaterials for tissue engineering applications.

Ren et al., Chemical Engineering Journal (2019) 370: 1027-1038 describe aligned porous poly (1-lactic acid) (PLLA) electrospun fibrous membrane with a biomimetic surface for the rapid regeneration of cartilage tissue. The PLLA electrospun fibers aligned in a single direction and generated ellipse-shaped nanopores in situ onto the surface of the fibers. Subsequently, chondroitin sulfate (CS) was coated on the surface of the fibers using polydopamine (PDA) as an adhesive polymeric bridge.

In Huang et al., Acta Biomaterialia (2018) 71: 184-199, rhBMP-2 (recombinant human bone morphogenetic protein-2) was tethered on chondroitin sulfate (CS)-functionalized calcium phosphate cement (CPC) scaffolds. CS was covalently conjugated onto the CPC scaffolds with the assistance of polydopamine (PDA) to form CPC-PDA-CS scaffolds.

However, membranes for hemodialysis, hemofiltration or hemodiafiltration, specifically polysulfone, polyethersulfone and polyarylethersulfon based membranes have so far not been considered as targets for modification with both polydopamine and chondroitin. It was now found that polydopamine and chondroitin can be used, in combination, for the coating of membranes comprising polysulfone, polyethersulfone or polyarylethersulfone and polyvinylpyrrolidone either in combination or as separate, consecutive layers with an unexpected benefit. The resulting polydopamine-chondroitin (PD-CS) coating is stable during a typical hemodialysis treatment and improves the performance and selectivity of the base membrane. Adding heparin to the coating either in combination with polydopamine and chondroitin or as another, separate layer, adds not only antithrombogenic properties, but also remains stable and supports said improved selectivity of the coated membrane.

So far, coatings have not been considered as a means to improve the selectivity of hemodialysis membranes. It was now found that the selectivity of dialysis membranes, specifically those based on polysulfone, polyethersulfone or polyarylethersulfone and polyvinylpyrrolidone, can be enhanced with a coating of polydopamine and chondroitin (PD-CS), optionally in combination with heparin (HEP) as another coating component (PD-CS-HEP). Such PD-PS or PD-CS-HEP coatings and their use for improving the selectivity of a membrane have so far not been described in the art.

## Summary

It is an object of the present invention to provide hollow fiber and flat sheet membranes for use in blood treatment applications and specifically in hemodialysis, hemofiltration or hemodiafiltration which are coated with a combination of polydopamine, chondroitin and, optionally, heparin. As used herein, the expression "membrane" or "membranes" encompasses both hollow fiber membranes and flat sheet membranes. In the context of the present invention, hollow fiber membranes are preferred as they constitute the state-of-the-art membrane configuration for use in hemodialysis devices.

Membranes that are preferably used as base membranes comprise a blend of i) a polysulfone, a polyethersulfone or a polyarylethersulfone and ii) polyvinylpyrrolidone. Said membranes can have different physical structures, including varying degrees of asymmetric configuration, ranging from minimum asymmetry in sponge-like structures to maximum asymmetry in finger-like structures, such as described, for example, in Ronco and Clark, Nature Reviews. Nephrology 14 (6) (2018) : 394-410). In case of hollow fiber membranes, the lumen or inner side of the hollow fibers membranes is preferably coated as it generally provides for the selective layer of the hollow fiber membranes that is in contact with blood, or, optionally, with blood plasma. In devices where the outer side of a hollow fiber membrane is in contact with blood and provides for the selective layer of the membrane, a modification or coating according to the invention would

preferably be applied to the outer side of the hollow fiber membranes.

According to one aspect, the base membrane is a MCO membrane and has a MWRO of from 8.5 to 14.0 kDa and a MWCO of from 50.0 to 130.0 kDa as measured by dextran sieving before blood contact, preferably a MWRO of from 8.5 to 12.5 kDa and a MWCO of from 55.0 to 110.0 kDa as measured by dextran sieving before blood contact. According to yet another aspect, the base membranes have a MWRO of from 9.0 to 12.5 kDa and a MWCO of from 55.0 to 90.0 kDa as measured by dextran sieving before blood contact. Such membranes are generally referred to as MCO membranes, sometimes also as HRO membranes. They are described in detail in WO 2015/118045 A1 and WO 2015/118046 A1, respectively. A product which is one representative of such MCO type membrane and dialyzer, and which is available in the market today is the Theranova dialyzer (Baxter).

According to another aspect, the base membrane is a HCO membrane having a MWRO of from 15kDa to 20kDa and a MWCO of from 170kDa to 320kDa as measured by dextran sieving before blood contact. Such membranes are described, for example, in WO 2004/056460 A1 or in Gondouin and Hutchison: High Cut-Off Dialysis Membranes: Current Uses and Future Potential. Advances in Chronic Kidney Disease 18 (2011):180-187. A product which is one representative of such HCO type membranes and dialyzers, and which is available in the market today is the Theralite dialyzer (Baxter). HCO membranes are characterized by a higher average pore size compared to the above-described MCO membranes, the mean pore size of which is lower, whereas the pore size distribution in MCO membranes is narrower compared to HCO membranes.

According to another aspect, the base membrane is a high-flux (HF) membrane having a MWRO of from 5.0 kDa to 8.5 kDa and a MWCO of from 25 kDa to 50 kDa as measured by dextran sieving before blood contact.

According to yet another aspect of the invention, the membranes can be coated in one step with a solution comprising dopamine, chondroitin and, optionally, heparin. For the avoidance of doubt, dopamine is the starting material which is provided in the solution for coating the base membrane and wherein it reacts to polydopamine. Accordingly, as used herein, dopamine refers to the starting material added to the coating solution but implies that it is transferred into polydopamine in the process of coating and is present as polydopamine on the final membrane.

According to one aspect, the concentration of dopamine in the coating solution is between 2 mg/mL to 80 mg/mL. The pH of the solution is preferably between 8.0 and 10, such as between 8.0 and 9.8, between 8.0 and 9.6, or between 8.2 and 9.4.

According to another aspect, the membranes are coated with polydopamine in an amount of from 0.30 $\mu g/cm^2$ to 1.10 $\mu g/cm^2$ of the membrane surface.

According to a further aspect, the membranes are coated with polydopamine and chondroitin in two consecutive steps, wherein the membranes are first coated with polydopamine followed by coating with chondroitin, thereby providing for coated membranes comprising or consisting of a base membrane which is coated with a first coating layer comprising or essentially consisting of polydopamine, and a second coating layer comprising or essentially consisting of chondroitin.

According to another aspect, the membranes are coated with polydopamine, chondroitin and optionally heparin in one step, wherein a mixture of polydopamine, chondroitin, and optionally heparin is applied to the membranes, thereby providing for a coated membrane comprising or essentially consisting of a base membrane which is coated with one essentially homogenous coating layer which comprises or essentially consists of a mixture of polydopamine and chondroitin.

According to another aspect, the membrane is coated with polydopamine, chondroitin and heparin in two or three consecutive steps, wherein the base membrane is first coated with polydopamine, resulting in a first coating layer essentially consisting of polydopamine, followed by coating with chondroitin in a second step, resulting in a second coating layer that essentially consists of chondroitin. Heparin can be applied in a third step. Resulting in a third coating layer that essentially consists of heparin. Alternatively, chondroitin and heparin are admixed and the polydopamine coated base membrane is coated with a combination of chondroitin and heparin to form a second coating layer that comprises heparin and chondroitin. Heparin can also be applied to the polydopamine coated base membrane in a second step and chondroitin can be applied in a third step, so the sequence of chondroitin and heparin in the respective layers is converted.

According to another aspect, the membranes are modified with polydopamine, chondroitin and optionally heparin by in situ modification of the base membrane with polydopamine in a first step **(Figure 2C),** followed by coating of the polydopamine modified base membrane with chondroitin and optionally heparin in a second step or consecutively in a second and third step in either sequence as described before.

Polydopamine can also be added according to any of the above processes to flat sheet membranes, i.e., either by an *in situ* casting process or by a one-step coating or a by consecutive coating steps as described above.

According to another aspect, the concentration of heparin in the coating solution is between 0.1 mg/mL to 100 mg/mL.

According to another aspect, the membrane is coated with heparin in an amount of from 5 $mU/cm^2$ to 100 $mU/cm^2$ of the membrane surface.

## Brief Description of the Drawings

Figure 1 is a schematic representation of sieving curves of hypothetic membranes having different selectivities as illustrated by their respective MWRO and MWCO values. When the difference between the molecular weight retention onset (MWRO) and molecular weight cut-off (MWCO) of a membrane decreases, the profile of the curve becomes steeper which corresponds to an increased selectivity. Starting from Membrane 1, this means that, for example, the MWCO can remain the same for both membranes (MWCO1 and MWCO2), while the MWRO is shifted to higher molecular weights, resulting in MWRO2 and in a Membrane 2 that has an increased selectivity. If the MWRO is shifted to higher molecular weights and the MWCO is shifted to lower molecular weights, the resulting Membrane 3 would have an even higher MWRO (MWRO3) combined with a lower MWCO (MWCO3), resulting in Membrane 3 having a further increased selectivity. It would also be possible to keep the MWRO3 at the same level as MWRO2 and only shift the MWCO3 to lower molecular weights (not shown here), which would also result in an improved selectivity versus Membrane 1. In consequence, methods for modulating the MWRO and MWCO of a given membrane mean that the removal of small, medium, and large middle molecules can be improved while the loss of albumin and other proteins in this molecular weight range and above can be decreased to achieve a better selectivity.

Figure 2 is a schematic representation of various processes that can be applied to modify a given membrane or membrane material with polydopamine, chondroitin and optionally heparin. FIG. 2(0) shows a finished hollow fiber membrane ("base membrane") without any polydopamine, chondroitin or heparin modification. Modification can be done successively by coating the hollow fiber membrane in a first step with polydopamine followed by coating with chondroitin in a second step (FIG. 2(A)), optionally followed by coating with heparin in a third step (not shown). The second and third step could, alternatively, also be combined and coating be done with a mixture of chondroitin and heparin. Alternatively, the coating can be done in one step with a coating solution comprising both (poly)dopamine and chondroitin (FIG. 2(B)). Alternatively, the coating solution could further contain heparin (not shown). Another option is shown in FIG. 2(C), where the membrane is modified in situ with polydopamine during spinning by adding it to the polymer. Chondroitin and optionally heparin can then be coated in one step onto the membrane or chondroitin and heparin can be applied to the membrane successively in either sequence (not shown).

Figure 3 is a schematic representation of how the coating of the invention is hypothesized to improve the selectivity of a membrane. FIG. 3(A) shows an unmodified membrane. Negatively charged molecules such as, for example, albumin can just pass through a membrane pore of appropriate size. FIG. 3(B) shows a membrane which is coated with polydopamine (PD) and heparin (HEP) whereby the membrane and pore receive a negatively charged layer and therefore reject passage of the same molecule which would have otherwise passed the pore. Another or additional effect that may contribute to an increase of rejection after modification is shown in FIG. 3(C). By reducing the pore size of larger pores more than smaller ones the selectivity of the overall membrane can also be increased.

Figure 4 is a schematic representation of a process for coating hollow fiber membranes in a filter module, such as a minimodule, in recirculation mode, whereby the coating solution is pumped through the filter or minimodule. This approach can be applied both for a one-step coating or successive coating.

Figure 5 shows the impact of the coating and the sterilization with either gamma rays or e-beam on the inhibition of cell growth and thus on the biocompatibility of the coating and sterilization method, respectively.

Figure 6 provides for a graphic presentation of heparin activity in polydopamine-chondroitin-heparin coated filter modules after e-beam sterilization.

Figure 7 shows the impact of coating a base membrane with polydopamine and chondroitin (triangles) on C3a activation in comparison with an uncoated membrane (circles) and a positive control (squares) which consists of a regenerated cellulose membrane (GFE18).

## Detailed Description

The expression "selectivity" as used herein for a hemodialysis membrane refers to the membrane’s ability to efficiently remove small (0.5-15kDa), medium (>15-25kDa) and large (>25-58kDa) middle-molecular weight compounds (see also Rosner et al.: Classification of Uremic Toxins and Their Role in Kidney Failure. Clin J Am Soc Nephrol. 2021) while essentially retaining larger proteins such as, specifically, albumin (66.4 kDa). Such selectivity is described, for

example, by the steepness of the sieving curve (e.g., dextran sieving curve) of a membrane. Increased steepness of the sieving curve correlates to increased selectivity. Accordingly, selectivity of a membrane can be described by its MWRO (molecular weight retention onset), i.e., the molecular weight at which the sieving coefficient of the membrane is 0.9, and its MWCO (molecular weight cut-off), i.e., the molecular weight at which the sieving coefficient of the membrane is 0.1, as these parameters provide for the necessary information on the shape of a membrane's sieving curve and, accordingly, on the above-mentioned removal and retention capabilities or selectivity of the membrane. For example, two membranes may have the same MWCO while differing in their MWRO. The membrane having the lower MWRO, accordingly, is less effective in removing molecules of a molecular weight below the cut-off. Its sieving curve is less steep, and the membrane is less selective than the one having a higher MWRO that is, accordingly, closer to the MWCO of the membrane, resulting in a better removal of said middle molecules while the retention capabilities remain the same.

The expression "MCO membrane(s)" or "MCO dialyzer(s)" as used herein, which is sometimes interchangeably used with the expression "HRO membrane(s)" or "HRO dialyzer(s)" for "High Retention Onset" membrane(s) or dialyzer(s), refers to membranes and dialyzers such as described, for example, in WO 2015/118046 A1 and WO 2015/118046 A1, respectively, and wherein the membrane (s) preferably has (have) a MWRO of from 9 to 12.5 kDa and a MWCO of from 55 to 110 kDa as measured by dextran sieving before blood contact and as otherwise described in WO 2015/118046 A1. Preferably MCO membranes are further defined by a sieving coefficient for albumin ($Sc_{Alb}$(%)) of below 1 (<1) as measured in human plasma at QB=300ml/min and QUF=60ml/min; a sieving coefficient for β-2-microglobulin ($Sc_{\beta 2m}$ (%)) of equal to or more than 95 (≥95) as measured in human plasma at QB=300ml/min and QUF=60ml/min; and a sieving coefficient for YKL-40 ($SC_{YKL-40}$ (%)) of equal to or more than 30 (≥30) as measured in human plasma at QB=300ml/min and QUF=60ml/min. An MCO dialyzer as mentioned herein is preferably further characterized by a KUF (ml/h/mmHg) of equal to or higher than 20 (≥20) as measured according to ISO 8637; a β2m clearance $K_{\beta 2m}$ (ml/min)of higher than 80 (>80) as measured according to ISO 8637 with QB=300-400mL/min; QD=700mL/min; QUF=0-10mL/min; a YKL-40 clearance $K_{YKL-40}$ (ml/min) of equal to or higher than 20 (≥20) as determined in human plasma with QB=300ml/min; QD=500ml/min; QUF=10ml/min and preferably further has an albumin loss (g/4h in vivo treatment or simulated use correlated with in vivo use) of equal to or below 7 (≤7.0), more preferably of equal to or below 4 (≤4.0).

The expression "dopamine" as used herein refers to the compound 2-(3,4-dihydroxyphenyl)ethylamine of formula (I) and including commercially available forms thereof, such as, for example, dopamine-hydrochloride (2-(3,4-dihydroxyphenyl)ethylamine-hydrochloride) .

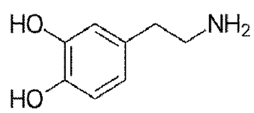

Formula (I)

The expression "polydopamine" (PD) refers to the polymer obtained from dopamine, for example by (auto)oxidation of dopamine in a basic medium (see, for example, Salomäki et al. J. Phys. Chem. B (2018), 122, 6314-6327. As used herein, the expression "polydopamine" encompasses various stages of polymerization starting from dopamine and, accordingly, dopamine and polydopamine may exist in various ratios during such process of polymerization.

The expression "chondroitin" as used herein refers to chondroitin sulfate (CS), a polymer with a wide molecular weight range composed of an alternating sequence of sulfated and/or unsulfated d-glucuronic acid (GlcA) and N-acetyl-d-galactosamine (GalNAc) residues linked through alternating β-(1 → 3) and β-(1 → 4) bonds and derived from various sources, wherein the polymer can be sulfonated at various positions. The expression encompasses chondroitin sulfates which are classified as CS-O,A,B,C,D,E,F,K (CS-A, chondroitin sulfate A, [GlcAβ1-3GalNAc(4 S)]; CS-C, chondroitin sulfate C, [GlcAβ1-3GalNAc(6 S)]; CS-D, chondroitin sulfate D, [GlcA(2 S)β1-3GalNAc(6 S)]; CS-E, chondroitin sulfate E, , [GlcAβ1-3GalNAc(4 S,6 S)]; CS-F, fucosylated chondroitin sulphate, [GlcA(α1-3Fuc)β1-3GalNAc(4 S)]; CS-O, chondroitin, [GlcAβ1-3GalNAc]) according to their sulfation pattern, as well as CS extracted from different sources. The expression further pertains to CS which may consist of combinations of different types or classes of CS. It preferably refers to the two major chondroitin sulfates A and C which differ from one another in the position of the sulfates. Chondroitin sulfate A is sulfated at position 4, and chondroitin sulfate C is sulfated at position 6.

The expression "heparin" as used herein refers to linear, unbranched, and highly sulfated polysaccharides belonging to the family of glycosaminoglycans (GAGs) and including (i) heparin and heparan sulfate, and (ii) hyaluronan. The expression "heparin" also encompasses the three forms of heparins which are available for therapeutic purposes, (i) unfractionated heparin (UFH), (ii) low molecular weight heparin (LMWH) and (iii) synthetic ultra (U)LMWH, see, for example, Laner-Plamberger et al., Int. J. Mol. Sci. 2021, 22, 12041.

The expression "base membrane" as used herein refers to a membrane before being coated with polydopamine, chondroitin and, optionally, heparin. In other words, the base membrane and the coated membrane only differ with regard to the presence of polydopamine, chondroitin and optionally heparin but is otherwise the same. Regarding *in situ* polydopamine modified membranes, the expression "base membrane" refers to a membrane that is essentially prepared

from the same polymer solution but does not contain polydopamine, and which is otherwise not coated with heparin and/or chondroitin.

The expression "blood treatment" as used herein refers to the treatment of whole blood or any blood product, such as, for example, blood plasma, of a human or animal patient. Such treatment comprises, but is not limited to, hemodialysis, hemofiltration or hemodiafiltration.

It was a goal of the present invention to provide membranes which are modified with polydopamine, chondroitin and, optionally, heparin. As used herein, the expression "modification" refers to both the *in situ* modification of a membrane with polydopamine followed by coating with chondroitin and optionally heparin, and to the coating of a membrane with polydopamine, chondroitin and optionally heparin. The membranes surprisingly show an increased selectivity versus membranes without such modification. In addition, they show an excellent hemocompatibility and antithrombogenicity, specifically when additionally modified with heparin. Such modification can be achieved in different ways according to the invention.

Membranes that can be used as base membranes according to the invention may be, for example, polysulfone (PSf), polyethersulfone (PES) or poly(aryl)ethersulfone (PAES) based membranes. They comprise a blend of i) a polysulfone, a polyethersulfone or a polyarylethersulfone and ii) polyvinylpyrrolidone. Base membranes according to the invention can also be made from other known materials as the concept of selectivity increase as described herein is connected to the modification of the pores of a given membrane. Other membrane materials that can be used are, for example, sulfonated polyacrylonitrile (PA or AN69), poly(methyl methacrylate) (PMMA), CTA (cellulose triacetate) or EVAL (ethylene vinyl alcohol copolymer). For an overview see, for example, Said et al., A Review of Commercial Developments and Recent Laboratory Research of Dialyzers and Membranes for Hemodialysis Application. Membranes 11, 767 (2021).

According to one aspect, base membranes according to the invention can have different physical structures, including varying degrees of asymmetric configuration, ranging from minimum asymmetry in sponge-like structures to maximum asymmetry in finger-like structures, such as described, for example, in Ronco and Clark, Nature Reviews. Nephrology 14 (6) (2018): 394-410). In case of hollow fiber membranes, the lumen or inner side of the hollow fibers membranes is preferably coated as it generally provides for the selective layer of the hollow fiber membranes that is in contact with blood, or, optionally, with blood plasma. In devices where the outer side of a hollow fiber membrane is in contact with blood and provides for the selective layer of the membrane, a modification or coating according to the invention would preferably be applied to the outer side of the hollow fiber membranes.

According to one aspect, the base membrane is a MCO membrane. A representative of such MCO membranes which is available in the market today is the Theranova dialyzer (Baxter) which comprises such MCO membrane.

According to another aspect, the base membrane has a MWRO of from 8.5 to 14.0 kDa and a MWCO of from 50.0 to 130.0 kDa as measured by dextran sieving before blood contact, preferably a MWRO of from 8.5 to 12.5 kDa and a MWCO of from 55.0 to 110.0 kDa as measured by dextran sieving before blood contact. According to yet another aspect, the base membranes have a MWRO of from 9.0 to 12.5 kDa and a MWCO of from 55.0 to 90.0 kDa as measured by dextran sieving before blood contact.

According to another aspect, the base membrane has MWRO of from 15kDa to 20kDa and a MWCO of from 170kDa to 320kDa as measured by dextran sieving before blood contact. Such membranes are described, for example, in WO 2004/056460 A1 or in Gondouin and Hutchison: High Cut-Off Dialysis Membranes: Current Uses and Future Potential. Advances in Chronic Kidney Disease 18 (2011):180-187. A representative of such HCO membranes available in the market today is the Theralite dialyzer (Baxter) which comprises such membrane.

According to another aspect, the base membrane is a high-flux (HF) membrane having a MWRO of from 5.0 kDa to 8.5 kDa and a MWCO of from 25 kDa to 50 kDa as measured by dextran sieving before blood contact.

According to one aspect of the invention, the membranes can be coated in one step with a coating solution comprising dopamine and chondroitin and, optionally, heparin. For the avoidance of doubt, dopamine is the starting material which is provided in the solution for coating the base membrane and wherein it reacts to polydopamine. Accordingly, as used herein, dopamine refers to the starting material added to the coating solution but implies that it is transferred into polydopamine in the process of coating and is present as polydopamine on the final membrane.

According to one aspect, the solution for coating the base membrane is prepared, for example, by providing a dopamine solution in a buffer, such as, for example, tris buffer, at an elevated pH (e.g., 8.0 to 9.5) to start the polymerization of dopamine. A color change is indicative for polydopamine formation. Chondroitin and optionally heparin are then added to the solution and dissolved therein. The solution can then be applied to, for example, the lumen of a hollow fiber membrane for the coating of the lumen of the membrane. For example, the coating solution can be filled into the lumen of the fibers by aspiration. The hollow fiber membrane can be incubated with the coating solution for a certain time which can be varied from about one minute to several hours. Generally, ten minutes to two hours of incubation time will be sufficient depending on the base membrane and the concentration of the dopamine and chondroitin in the solution. The lumen of the hollow fiber can then be rinsed, e.g., with 0.9% NaCl or distilled water. The flow rate during rinsing can be varied over a relatively broad range, e.g., from about 10 mL/min to 50 ml/min, and the rinsing time can also be varied from about 1 to 20 minutes, such as, for example, from about 2 to 10 minutes.

According to another aspect, hollow fiber membranes can be coated in recirculation mode, wherein the coating solution is provided in a pool from which it is pumped, for example, through the hollow fibers of a filter device (FIG. 4). The pool volume can vary, but about 80 to 500 mL of a coating solution will be sufficient for an effective coating of a standard dialyzer having a surface area of about 1.5 -2.2 $m^2$. The pump flow can also be varied over a relatively broad range, such as, for example, from 1 mL/min to about 20 mL/min. After coating the filters can be rinsed as described before.

The process provides for a coated membrane comprising a base membrane which is coated with one essentially homogenous coating layer which comprises or essentially consists of a mixture of polydopamine and chondroitin. Optionally, the process provides for a coated membrane comprising a base membrane which is coated, on its lumen side, with one essentially homogenous coating layer on top of said base membrane which comprises or essentially consists of a mixture of polydopamine, chondroitin and heparin.

According to one aspect, the concentration of dopamine in the coating solution is between 2 mg/mL to 80 mg/mL. According to another aspect of the invention the concentration of dopamine in the coating solution is between 3 mg/mL and 60 mg/mL. According to yet another aspect of the invention the concentration of dopamine in the coating solution is from 5 mg/mL to 25 mg/mL. According to yet another aspect of the invention the concentration of chondroitin in the coating solution is between 5 mg/mL and 80 mg/mL, such as between 10 mg/mL and 60 mg/mL.

According to another aspect, the membranes are coated with polydopamine in an amount of from 0.30 $\mu g/cm^2$ to 1.10 $\mu g/cm^2$ of the membrane surface. According to another aspect of the invention, the membranes are coated with chondroitin in an amount of from 0.50 $\mu g/cm^2$ to 1.20 $\mu g/cm^2$, such as 0.30 $\mu g/cm^2$, 0.40 $\mu g/cm^2$, 0.50 $\mu g/cm^2$, 0.60 $\mu g/cm^2$, 0.70 $\mu g/cm^2$, 0.80 $\mu g/cm^2$, 0.90 $\mu g/cm^2$, 1.00 $\mu g/cm^2$, 1.10 $\mu g/cm^2$, or 1.20 $\mu g/cm^2$. According to yet another aspect of the invention, the membranes are coated with polydopamine in an amount of from 0.40 $\mu g/cm^2$ to 0.60 $\mu g/cm^2$ of the membrane surface. According to yet another aspect of the invention, the membranes are coated with chondroitin in an amount of from 0.30 $\mu g/cm^2$ to 0.70 $\mu g/cm^2$.

According to yet another aspect of the invention the concentration of chondroitin in the coating solution is between 5 mg/mL and 80 mg/mL, preferably between 10 mg/mL and 60 mg/mL, such as, for example, between 10 mg/mL and 50 mg/mL .

According to one aspect of the invention, chondroitin sulfate A is used for coating the membranes. Chondroitin sulfate A is available in different molecular weights ranging from, for example, 4 kDa to 50 kDa. According to the invention, all chondroitin molecular weigh variants can be used effectively for the coating of a membrane. According to another aspect of the invention, chondroitin with a higher molecular weight, such as from about 10 kDa to 50 kDa is used for coating a membrane. According to yet another aspect of the invention, chondroitin with a lower molecular weight, such from about 4 kDa to 15 kDa is used for coating a membrane.

According to another aspect of the invention, the membranes are coated with chondroitin in an amount of from 0.30 $\mu g/cm^2$ to 1.20 $\mu g/cm^2$, such as 0.30 $\mu g/cm^2$, 0.40 $\mu g/cm^2$, 0.50 $\mu g/cm^2$, 0.60 $\mu g/cm^2$, 0.70 $\mu g/cm^2$, 0.80 $\mu g/cm^2$, 0.90 $\mu g/cm^2$, 1.00 $\mu g/cm^2$, 1.10 $\mu g/cm^2$, or 1.20 $\mu g/cm^2$. According to yet another aspect of the invention, the membranes are coated with polydopamine in an amount of from 0.40 $\mu g/cm^2$ to 0.60 $\mu g/cm^2$ or from 0.50 $\mu g/cm^2$ to 0.60 $\mu g/cm^2$ of the membrane surface. According to yet another aspect of the invention, the membranes are coated with chondroitin in an amount of from 0.60 $\mu g/cm^2$ to 1.10 $\mu g/cm^2$.

According to another aspect, the concentration of heparin in the coating solution is between 0.1 mg/mL to 100 mg/mL, from 0.5 mg/mL to 80.0 mg/mL, from 1 mg/mL to 60 mg/L, or from 5 mg/mL to 40 mg/L. For example, the concentration of heparin in the coating solution is 0.5 mg/mL, 1 mg/mL, 2 mg/mL, 3 mg/mL, 4 mg/mL, 5 mg/ml, 10 mg/ml, 15 mg/ml, 20 mg/ml, 25 mg/ml, 30 mg/ml, 35 mg/ml, 40 mg/ml, 45 mg/ml, 50 mg/ml, 55 mg/ml, 60 mg/ml, 65 mg/ml, 70 mg/ml, 75 mg/ml, or 80 mg/ml. According to a further aspect, the concentration of heparin in the coating solution is between 10 mg/mL and 50 mg/mL. According to a further aspect, the concentration of heparin in the coating solution is between 0.1 mg/mL to 20 mg/mL, from 0.1 mg/mL to 10 mg/mL or from 0.1 mg/mL to 10 mg/mL.

According to another aspect, the membrane is coated with heparin in an amount of from 5 $mU/cm^2$ to 100 $mU/cm^2$ of the membrane surface, from 5 $mU/cm^2$ to 80 $mU/cm^2$ of the membrane surface, from 5 $mU/cm^2$ to 50 $mU/cm^2$ of the membrane surface, or from 5 $mU/cm^2$ to 30 $mU/cm^2$ of the membrane surface, such as, for example 5 $mU/cm^2$, 6 $mU/cm^2$, 7 $mU/cm^2$, 8 $mU/cm^2$, 9 $mU/cm^2$, 10 $mU/cm^2$, 11 $mU/cm^2$, 12 $mU/cm^2$, 13 $mU/cm^2$, 14 $mU/cm^2$, 15 $mU/cm^2$, 16 $mU/cm^2$, 17 $mU/cm^2$, 18 $mU/cm^2$, 19 $mU/cm^2$, 20 $mU/cm^2$, 21 $mU/cm^2$, 22 $mU/cm^2$, 23 $mU/cm^2$, 24 $mU/cm^2$, 25 $mU/cm^2$, 30 $mU/cm^2$, 35 $mU/cm^2$, 40 $mU/cm^2$, 45 $mU/cm^2$, 50 $mU/cm^2$, 55 $mU/cm^2$, 60 $mU/cm^2$, 65 $mU/cm^2$, 70 $mU/cm^2$, 75 $mU/cm^2$, or 80 $mU/cm^2$. For example, the membrane is coated with heparin in an amount of from 5 $mU/cm^2$ to 25 $mU/cm^2$ of the membrane surface.

The use of heparin for coating a membrane according to the invention may additionally reduce the thrombogenicity of the membranes (**Examples 8 and 9, Figure 6**). It was a surprising finding that the use of heparin as an additional component in a polydopamine-chondroitin coating can still impart an antithrombogenic function without interfering with the selectivity increasing effect of the polydopamine-chondroitin coating, and that this effect is maintained also after sterilization. Heparin even seems to contribute to the selectivity-increasing effect of the coating.

According to another aspect, the base membranes are coated with polydopamine and chondroitin or with

polydopamine, chondroitin and heparin in two or three consecutive steps **(Figure 2A),** wherein the membranes in a first step are coated with polydopamine, followed in a second step by coating with chondroitin **(Figure 2A),** optionally followed in a third step by coating with heparin. Alternatively, chondroitin and heparin can be applied to the polydopamine coated membrane in one step. Said process provides for coated membranes comprising a base membrane which is coated with a first essentially homogenous coating layer comprising or essentially consisting of polydopamine, a second essentially homogenous coating layer comprising or essentially consisting of chondroitin and optionally heparin, and optionally a third layer comprising or essentially consisting of heparin, where the second layer only comprises or essentially consists of chondroitin.

According to another aspect, the membranes are modified with polydopamine by *in situ* modification of the base membrane with polydopamine in a first step **(Figure 2C)**, followed by coating of the *in situ* polydopamine modified base membrane with heparin and/or chondroitin in a second and optionally third step as described above. In situ modification with polydopamine can be achieved by directly including dopamine into the spinning solution as described in **Example 2.3.** The concentration of dopamine in the spinning solution can be varied over a certain range, such as, for example, from about 0.3 to 0.8 wt.%, relative to the total weight of the polymer solution, such as from about 0.4 wt.% to 0.6 wt.%. Dopamine can either be added to the spinning solution after all ingredients except dopamine have been mixed until homogeneity, or can immediately be added to the other ingredients for preparing the spinning solution. Polydopamine is formed upon spinning within the membrane of which it is then an integral part. The finished, polydopamine modified membrane can then be coated with heparin and/or chondroitin as described above in a one-step coating or a subsequent coating approach.

Polydopamine can also be coated on or added to a flat sheet base membrane according to any of the above processes, i.e., either by an *in situ* casting process or by coating with polydopamine as described there, followed by coating with heparin and/or chondroitin as described above in a one-step coating or a subsequent coating approach.

The presence and amount of polydopamine and chondroitin on the finished fibers can be tested with known assays such as a protein assay based on bicinchoninic acid for polydopamine (Smith et al., Analytical Biochemistry 150: 76-85 (1985)). For quantification of chondroitin, a dimethylmethylene blue (DMMB) assay can be used (Farndale et al., Biochimica et Biophysica Acta 883: 173-177 (1986)).

The membranes or complete filters that have been modified or coated according to the invention, can be sterilized either with steam sterilization, e-beam sterilization at a dose of from about 25 to 30 kGy or gamma sterilization at a dose of from about 25 to 30 kGy. Gamma sterilization can be done after drying with pressurized air and optionally filling with 2 grams of residual water prior to sterilization, or with water filled filter devices, optionally in the presence of an oxygen scavenger. Alternatively, e-beam sterilization can be used for sterilization which well maintains the integrity and stability of the coating and the performance of the coated hollow fibers and filter device. According to one aspect, e-beam and gamma ray sterilization is used in connection with modified and/or coated membranes according to the invention. According to yet another aspect, e-beam sterilization is used in connection with modified and/or coated membranes according to the invention.

According to one aspect of the invention, the above-described modification and/or coating of a given membrane, such as a polysulfone, polyarylethersulfone or polyethersulfone based membrane, with polydopamine and chondroitin or with polydopamine, chondroitin and heparin has an unexpected influence on the sieving performance and in consequence, on the selectivity of the membrane. Marker compounds that can be used for assessing said influence by determining their respective sieving coefficients are advantageously selected over a range of different molecular weight and include, for example, compounds representative for small middle molecules, medium middle molecules, large middle molecules and large molecules as defined above. Examples are, accordingly, β2-M, myoglobin, complement factor D, YKL-40. In addition, albumin is used as another marker molecule. For determining the effect of the coating according to the invention on the sieving performance and selectivity of a given base membrane, sieving coefficients for selected marker molecules can be determined before and after the modification or coating with polydopamine, chondroitin and, optionally, heparin. Said sieving coefficients can then be compared. As explained with a view on MWRO and MWCO in **Figure 1,** a similar assessment can be made based on, for example, the sieving coefficients of the molecules with a lower and a higher molecular weight, respectively. For example, maintaining the sieving coefficient of a lower molecular weight molecule, such as, for example, YKL-40, while at the same time the sieving coefficient for albumin can be decreased, indicates an increase in selectivity as the membrane gets less permeable for albumin whereas it retains its desired permeability for larger middle molecules. Comparisons of uncoated or unmodified membranes and membranes coated with polydopamine, heparin and/or chondroitin are provided in **Example 4, Tables II, III** and **IV** for different membranes.

According to another aspect of the invention, the modified or coated membranes according to the invention are highly hemocompatible and they maintain their hemocompatibility also after sterilization especially in case of e-beam sterilization **(Figure 5**). According to one aspect of the invention, the modified and/or coated membranes can be used as membranes in medical applications, including for applications that require a direct contact of the coated surface with the blood or blood components of a patient. For example, modified and/or coated membranes according to the invention

can be used for preparing dialyzers for use in hemodialysis, including chronic and acute (CRRT) applications and encompassing hemodiafiltration and hemofiltration.

According to another aspect of the invention, the modified or coated membranes, where the coating comprises heparin, have a reduced antithrombogenic activity and they maintain such antithrombogenicity also after sterilization especially in case of e-beam sterilization **(Figure 6).**

According to another aspect of the invention, filters or hemodialyzers comprising modified or coated membranes according to the invention have an excellent clearance **(Example 5)**.

According to yet another aspect of the invention, filters or hemodialyzers are provided that comprise hollow fiber membranes that have been modified and/or coated according to the invention on the lumen side or on the outer surface of the hollow fiber membranes. According to one aspect, said filters or hemodialyzers comprise hollow fiber membranes which are coated with polydopamine and chondroitin. According to another aspect, said filters or hemodialyzers comprise hollow fiber membranes which are coated with polydopamine, chondroitin and heparin. According to yet another aspect, said filters or hemodialyzers comprise hollow fiber membranes wherein the membranes are modified *in situ* with polydopamine and are coated with chondroitin or with a combination of chondroitin and heparin.

According to another aspect of the invention, the filters and dialyzers of the invention that comprise hollow fiber membranes which are modified and/or coated with polydopamine, heparin and/or chondroitin can be used in extracorporeal blood purification or extracorporeal blood treatment, including, for example, hemodialysis, hemodiafiltration and hemofiltration.

It will be readily apparent to one skilled in the art that various substitutions and modifications may be made to the invention disclosed herein without departing from the scope and spirit of the invention.

The present invention will now be illustrated by way of non-limiting examples to further facilitate the understanding of the invention.

## Examples

### Example 1: Basic Materials and Methods

#### Example 1.1: Preparation of minimodules

Minimodules (fibers in a housing used as standardized filters for analysis) are prepared by cutting fibers to a length of 20 cm, drying the fibers for 1 h at 40°C and <100 mbar and subsequently transferring the fibers into the housing. The ends of the fibers are potted with polyurethane. After the polyurethane has hardened, the ends of the potted membrane bundle are cut to reopen the fibers. The minimodule ensures protection of the fibers. In the present examples, minimodules having an effective membrane (lumen) surface A of 360 $cm^2$ were used. The number of fibers required for an effective surface A of 360 $cm^2$ is calculated according to equation (1)

$$A=\pi\times d_i\times l\times n\,[cm2] \quad (1),$$

wherein di is the inner diameter of fiber [cm], n represents the number of fibers, and 1 represents the effective fiber length [cm]. Each minimodule has an effective length of approx. 170 mm (without PU potting) and an inner diameter of 10 mm. The internal diameter of fibers and the wall thickness depends on the specific membranes used. Hence, the packing density generally varies between 23% and 31%.

#### Example 1.2: Hydraulic Permeability (Lp) of minimodules

The hydraulic permeability of a minimodule is determined by pressing a defined volume of water under pressure through the minimodule, which has been sealed on one side, and measuring the required time. The hydraulic permeability is calculated from the determined time t, the effective membrane surface area A, the applied pressure p and the volume of water pressed through the membrane V, according to equation (2):

$$Lp = V / [p \cdot A \cdot t] \quad (2)$$

The effective membrane surface area A is calculated from the fiber length and the inner diameter of the fiber according to equation (3)

$$A = \pi \cdot d_i \cdot 1 \cdot [cm^2] \quad (3)$$

wherein di represents the inner diameter of fiber [cm] and 1 represents the effective fiber length [cm].

The mini-module is wetted thirty minutes before the Lp-test is performed. For this purpose, the minimodule is put in a box containing 500 mL of ultrapure water. After 30 minutes, the minimodule is transferred into the testing system. The testing system consists of a water bath that is maintained at 37°C and a device where the minimodule can be mounted. The filling height of the water bath has to ensure that the minimodule is located underneath the water surface in the designated device. In order to avoid that a leakage of the membrane leads to a wrong test result, an integrity test of the minimodule and the test system is carried out in advance. The integrity test is performed by pressing air through the minimodule that is closed on one side. Air bubbles indicate a leakage of the minimodule or the test device. It must be checked if the leakage is due to an incorrect mounting of the minimodule in the test device or if the membrane leaks. The minimodule must be discarded if a leakage of the membrane is detected. The pressure applied in the integrity test must be at least the same value as the pressure applied during the determination of the hydraulic permeability in order to ensure that no leakage can occur during the measurement of the hydraulic permeability because the pressure applied is too high.

**Example 1.3: Dextran sieving measurements**

**Example 1.3.1: Dextran solutions**

Fractions of dextran supplied by Fluka (Mw 6, 15-20, 40, 70, 100, 200, 500 kDa) and Sigma-Aldrich (Mw 9-11 kDa) (both from Sigma-Aldrich Co. LLC, St. Louis, USA) are used without further purification. Solutions of dextrans with the different molecular weight fractions are combined in Millipore water (i.e., Type 1 ultrapure water, as defined by ISO 3696) at a concentration of 1 g/l for each fraction, which results in an overall concentration of 8 g/l.

**Example 1.3.2: Dextran sieving coefficient tests**

Filtration experiments were carried out under a constant shear rate ($\gamma$=750s$^{-1}$) and with the ultrafiltration rate set at 20% of the blood side entrance flux $Q_{Bin}$, calculated according to equation (4):

$$Q_{Bin} = \frac{\gamma \cdot n \cdot \pi \cdot d_i^3 \cdot 60}{32} \quad (4),$$

where $Q_{Bin}$ is the flux at the blood side entrance in ml/min; n is the number of fibers in the minimodule; $d_i$ is the inner diameter of the fibers in cm and $\gamma$ is the constant shear rate mentioned above. The filtration conditions are without backfiltration, contrary to the conditions typical of hemodialysis. The dextran solution was recirculated at 37°C ± 1°C. Feed (blood side entrance), retentate (blood side exit), and filtrate (dialysate exit) samples were taken after 15 min. Relative concentrations and molecular weights of the samples were analyzed via gel permeation chromatography. The analysis was carried out in a High Performance Liquid Chromatography (HPLC) device (HP 1090A or Agilent 1200; Agilent, Santa Clara, CA, USA) equipped with an RI detector (G1362 from Agilent) and TSKgel columns (PWXL-Guard Column, G 3000 PWXL, G 4000 PWXL; Tosoh, Tessenderlo, Belgium). Samples were filtered through a 0.45 μm filter type OE67 from Schleicher and Schnell, Einbeck, Germany. Calibration was done against dextran standards (Fluka). The sieving coefficient SC is calculated according to equation (5)

$$SC = \frac{2 \cdot c_F}{c_p + c_R} \quad (5),$$

wherein $C_F$ is the concentration of the solute in the filtrate, $C_P$ its concentration in the permeate and $C_R$ its concentration in the retentate.

**Example 1.4: Measurement of sieving coefficients in human plasma**

Middle molecules, consisting mostly of peptides and small proteins with molecular weights in the range of

500-60,000 Da, accumulate in renal failure and contribute to the uremic toxic state. Beta2-microglobulin (beta2-MG or β2-M) with a molecular weight of 11 kDa is considered one of the smaller representatives of these middle molecules. Myoglobin has a molecular weight (MW) of about 17 kDa is already larger. It will not be completely cleared from blood by known high-flux dialyzers, whereas it is readily removed by high cut-off dialyzers. Complement factor D is a serine protease with a molecular weight of 25 kDa and is a good candidate for assessing the sieving coefficient for a molecule in the medium molecular weight range. YKL-40 with a molecular weight of 40 kDa is considered a representative of higher molecular weight middle molecules that should still be removed as efficient as possible. Albumin with a MW of about 67 kDa is a key element in describing the sieving characteristics of membranes, as albumin should preferably not be allowed to pass a membrane for chronic hemodialysis to a significant extent, whereas molecules having a lower molecular weight, such as β2-M and YKL-40 should be removed as efficiently as possible, i.e., the sieving curve of a given membrane should be steep and not flat, as is typically found with protein-leaking membranes.

The sieving coefficients for albumin and YKL-40 which are naturally contained in human plasma can be determined for uncoated membranes and membranes that were coated according to the invention or, for comparison, coated with dopamine or chondroitin only. Determination of sieving coefficients for these marker molecules was done with Octaplas LG from Octapharma. The respective minimodules as described in **Example 1.1** and coated as described in **Example 2** and in **Example 4** were used.

For the experiments, human plasma was thawed and brought to 37°C under careful stirring. Other marker substances can then be added to the plasma as required. For each minimodule 150g of the human plasma was used. The final protein concentration of the test solution was 45±5 g/l. The minimodules were rinsed with 40 mL 0.9% NaCl solution on the blood and dialysate side. Then the blood and dialysate sides are blown out at 0.3 ± 0.1 bar for 10 ± 5 s. $Q_B$ was 8,7 mL/min (= 7,71 SKT) and $Q_{UF}$ was 1,7 mL/min (=1,58 SKT). The minimodules were connected to a new tubing set with the open filtrate side on top. The recirculating tubes were returned to the solution reservoir. The blood-in pump was started. When the minimodules were free of air bubbles, the filtrate pump was started. As soon as filtrate flows back from the tube into the pool time was started. After 30 minutes test time a sample of the test solution (A) (minimum 1,1 mL for HSA and 550 μL for YKL-40) was taken. The sampling time was 1 min for $Q_{UF}$ and 1 min for $Q_{Bout}$. From these measurements, the retentate sample (R) and filtrate sample (F) are used for further analysis of the marker molecules. The sieving coefficient SC is calculated according to equation (5).

**Example 1.5: Clearance Performance**

The clearance "C" (mL/min) refers to the volume of a solution from which a solute is completely removed per unit time. In contrast to the sieving coefficient which is the best way to describe a membrane as the essential component of a hemodialyzer, clearance is a measure of the overall dialyzer function and hence dialysis effectiveness. Accordingly, the coating as described before was applied to complete hemodialyzers, such as commercially available dialyzers like Theranova 400 (Baxter Int., Inc.) or hemodialyzers were prepared from other MCO type membranes, such as the MCO-Ci 400 dialyzer (Gambro Dialysatoren GmbH, Hechingen, Germany) or the MCO 3 prototype (Gambro Dialysatoren GmbH, Hechingen Germany; see, for example, Boschetti-de-Fierro et al. (2015), Scientific Reports 5: 18448, DOI: 10.1038/srep18448) and were coated according to the invention. If not indicated otherwise, the clearance performance of a dialyzer was determined according to ISO 8637:2004(E). The set-up of the test circuit was as shown in ISO 8637:2004(E). Flows are operated in single path.

To measure clearance, human plasma (protein content 55 ± 10 g/L) is spiked with myoglobin and β2-M (Lee Biosolutions), to a concentration of 0.50 mg/L and 5.0 mg/L , respectively. Albumin, complement factor D and YKL-40 are measured directly from plasma concentrations. Samples are in each case taken from the blood outlet, dialysate outlet and blood inlet, respectively. A first sample is taken after 4 minutes.

**Example 1.6: Quantitative determination of YKL-40 and complement factor D in human plasma**

For determining the concentration of YKL-40 and human complement factor D the Quantikine Human Chitinase 3-like (CHI3L1) and Quantikine ELISA Human Complement Factor D (CFD) ELISA Immunoassay (R&D Systems Europe, Ltd.), respectively, are used according to the supplier's instructions. The assays are a 4.5- and 3.5-hour solid phase ELISA designed to measure CHI3L1 and CFD in cell culture supernates, serum, plasma, and urine. Both assays employ the quantitative sandwich enzyme immunoassay technique. A monoclonal antibody specific for the analyte has been pre-coated onto a microplate. Standards and samples are pipetted into the wells and any analyte present is bound by the immobilized antibody. After washing away any unbound substances, an enzyme-linked polyclonal antibody specific for the analyte is added to the wells. Following a wash to remove any unbound antibody-enzyme reagent, a substrate solution is added to the wells and color develops in proportion to the amount of CHI3L1 or CFD bound in the initial step. The color development is stopped, and the intensity of the color is measured.

For determining the concentration of β2-M, myoglobin and albumin a nephelometer (BN ProSpec, Siemens

Medical Solutions) is used in combination with the supplier's quantification kits. β2-M, myoglobin and albumin are quantified using the N Latex β2 Microglobulin kit, N Latex Myoglobin kit and N Antiserum to Human albumin kit (Siemens Health solutions), respectively, all according to the supplier's instructions. Each determinant is measured separately by formation of an insoluble antigenantibody complex upon addition of the provided antibody. The complex formation proceeds in excess of antibody, is measured using light scattering by the nephelometer and compared to standards of known concentration.

**Example 2: Coating of hollow fiber membranes with polydopamine, chondroitin and optionally heparin**

**Example 2.1: One-step coating with polydopamine and chondroitin**

A 6 mg/mL dopamine solution (600 mg dopamine hydrochloride from Sigma Aldrich in 100 mL of tris buffer (10 mmol/L, pH 8.6)) was prepared and the solution was stirred for 30 minutes to start the polymerization of dopamine. A color change is indicative for polydopamine formation. Then, 20 mg/mL of chondroitin (2000 mg chondroitin sulfate A sodium salt from bovine trachea (Sigma Aldrich) was added and the solution was stirred vigorously until the chondroitin was dissolved completely, which took about 5 minutes. The resulting solution was immediately used for a one-step coating by filling the lumen of the fibers of a minimodule from bottom to top by aspiration with a syringe. During filling the minimodule was shaken to remove air bubbles. The minimodule was then incubated for 1 hour at room temperature before the solution was removed. The minimodule was rinsed using 0.9% NaCl at a flowrate of 20 mL/min for 5 min on the lumen-side, followed by 5 min on the filtrate-side, followed by the repeat with Millipore water. After rinsing the minimodule was dried using compressed air for 80 minutes at 0,3 bar.

Alternatively, coating of filters or minimodules can be done in recirculation mode as shown schematically in **Figure 4,** which reduces the time required for coating compared to the static approach described above. In this case, minimodules or filters are coated on the lumen side of the fibers with a pump flow of, for example, 5 mL/min and a pool volume of about 100 mL for coating an average of four minimodules (25 mL per minimodule). During recirculation, the filtrate side of the minimodules or filters were closed with a connector. Concentrations of dopamine, chondroitin and heparin are as indicated above and/or in the respective Examples.

The minimodules, after coating, were tested the next day or sterilized first either with steam sterilization, e-beam sterilization at a dose of from about 25 to 30 kGy or gamma sterilization at about 25 to 30 kGy before testing. Gamma sterilization can be done after drying with pressurized air after coating and filling with 2 grams of residual water prior to sterilization or with water filled minimodules (or filters), optionally in the presence of an oxygen scavenger. Alternatively, e-beam sterilization can be used to maintain the integrity and stability of the coating and the performance of the coated hollow fibers and filter device.

In order to investigate the role of different chondroitin sulfate sources that provide chondroitin with different average molecular weights, analogous coating experiments were carried out with Mythochondro® chondroitin sulfate sodium (Gnosis S.p.A.)having an average molecular weight of from 4000 to 12000 Da, and with chondroitin sulfate A sodium salt (Carbosynth Ltd.) having an average molecular weight of from 10000 to 50000 Da. No MW is provided for chondroitin sulfate A sodium salt from Sigma Aldrich. The respective chondroitin variants were used as indicated in the respective Examples.

In addition to polydopamine and chondroitin in the one-step coating as described here, heparin (HEP) was added in a concentration to the coating solution in a concentration as disclosed herein. The coating protocol remained unchanged.

**Example 2.2: Sequential (successive) coating with polydopamine and chondroitin**

For a sequential coating of the hollow fiber membranes the same minimodules and solutions were used. However, the dopamine solution was applied to the lumen of the hollow fibers once the dopamine had started to polymerize without adding chondroitin, followed by incubation for 1 hour and subsequent rinsing and flushing as described above. In a second step, the polydopamine-coated membrane was submitted to coating with chondroitin, which was applied to the hollow fibers as described above, followed by incubation for another hour and subsequent rinsing and flushing. The coated minimodules were set aside for testing on the next day.

For comparison, minimodules were also used for coating with either polydopamine or with chondroitin as described above. The goal was to determine the respective influence of either compound on the selectivity of a given membrane in comparison to a membrane according to the invention which is coated with both polydopamine and chondroitin.

**Example 2.3: In situ modification with polydopamine**

Instead of modifying or functionalizing membranes such as described herein post-production by coating with polydopamine and chondroitin and, optionally, heparin, they can also be modified with polydopamine *in situ,* followed by coating with chondroitin or chondroitin and heparin together. For the membrane production, polyethersulfone (PES) (Ultrason 6020 P, BASF, Germany), polyvinylpyrrolidone K90 (PVP K90) (average molecular weight of 360 kDa, Carl Roth, Germany) and polyvinylpyrrolidone K30 (PVP K30) (average molecular weight of 40 kDa, Carl Roth, Germany) are purchased. N-methyl-2-pyrrolidone (NMP) (99 %, Fisher Scientific, Germany) functions as solvent for the membrane preparation. Dopamine hydrochloride (98 %, Sigma Aldrich, Germany) was used as functional ingredient. For the membrane post-treatment, glycerol was purchased at Carl Roth with a purity above 99 %. Tris-(hydroxymethyl)-aminomethan (tris) (≥99.9 %, Carl Roth, Germany), sodium acetate (≥99 %, Carl Roth, Germany) and hydrochloric acid (ACS reagent, Sigma Aldrich, Germany) were used as buffer solutions and to adjust the pH of the different solutions.

For the functionalization of membranes with polydopamine and heparin, solutions were prepared and used in different membrane functionalization procedures to achieve the 149 desired membrane structures. The polymer solution is summarized in **Table 0:**

***Table 0:*** *Overview of polymer solution compositions for flat sheet casting and hollow fiber spinning*

| Polymer Solution | PES **[wt.%]** | NMP **[wt.%]** | PVP K30 **[wt.%]** | PVP K90 **[wt.%]** | Dopamine **[wt.%]** |
|---|---|---|---|---|---|
| P1 | 22.0 | 78.0 | - | - | - |
| P2 | 21.9 | 77.6 | - | - | 0.5 |
| P3 | 15.9 | 75.6 | 4 | 4 | 0.5 |
| P4 | 14.0 | 84.0 | - | 2 | - |
| P5 | 14.0 | 83.5 | - | 2 | 0.5 |
| P6 | 14.0 | 85.5 | - | - | 0.5 |

For the polymer solutions P1 to P4, all ingredients were stirred with a KPG stirrer at room temperature for at least 24 h until a homogeneous solution was formed. Before membrane fabrication, the solutions were left for degassing overnight to avoid defects in the membranes. For the polymer solutions P5 and P6, all ingredients except dopamine were stirred with a KPG stirrer at 65°C for at least 24 h until a homogeneous solution was formed. 2 h before transforming the polymer solutions into membranes, dopamine was added into the polymer solution and stirred until a homogeneous solution was formed. Subsequently, the solutions were left degassing around 2 h.

The bore solution consisted of NMP (50 wt.%) and $H_2O$ (wt.%). Alternatively, tris buffer (10 mM, 50 wt.%) at a pH of, for example, 8.5, can be used instead of $H_2O$. For fiber fabrication, a conventional spinning setup is used. The spinning parameters were as follows: Polymer solution flow rate [g/min] was 6.8, the polymer solution temperature [°C] was 65°. The Bore Flow rate [mL/min) was 10.3. The air gap was 2cm and the coagulation bath temperature was 65°C. During spinning, the polymer and the bore solution passed through a double orifice spinneret with a diameter of 0.4 mm for the bore and 1.12 mm for the polymer solution. The fiber emerged from the spinneret, passed through the air gap and entered the coagulation bath. The coagulation bath consisted of DI water. The take-up speed was controlled by a motorized pulling wheel, which conveys the fiber into a washing bath. Then, the fibers were stored in DI water for 34 h before storing them for another 24 h in a 5 wt.% glycerol in DI water solution. Finally, the fibers are air dried at room temperature overnight before further use.

**Example 3**: **Quantification of polydopamine and chondroitin on membrane fibers**

The amount of polydopamine and of chondroitin on polyethersulfone (PES) fibers after coating were determined by placing fibers in 2 mL cryovials and adding the respective detection reagents for each substance. For the quantification of polydopamine, a known protein assay based on bicinchoninic acid was used (Smith et al., Analytical Biochemistry (1985) 150: 76-85). For quantification of chondroitin, a dimethylmethylene blue (DMMB) assay was used (Farndale et al., Biochimica et Biophysica Acta (1986) 883: 173-177).

Both methods were utilized using a 200 pg/mL-1 stock solution, of which 100µl (polydopamine) or 200 µl (chondroitin) were combined with 2mL of reagent. Since solid membranes were used, the dilution of the stock solution in the reagent was utilized to calculate the final standard concentration and this number was then used to determine the respective quantities on membrane the fibers. In this experiment, hollow fiber membranes based on polyethersulfone were coated with a solution of dopamine/polydopamine and chondroitin with concentrations of 12 mg/mL dopamine and

40 mg/mL chondroitin. The samples were prepared in line with **Example 1** and the presence of polydopamine and chondroitin on the final membrane was determined. Quantities were determined in parallel assays based on 1, 5 and 10 fibers, respectively **(Table I**).

**Table I**: *Average amount of polydopamine and chondroitin found on PES hollow fiber membranes coated with 12 mg/mL dopamine and 40 mg/mL chondroitin.*

| | Polydopamine (μg/cm²) | Chondroitin (μg/cm²) |
|---|---|---|
| Based on # of fibers | Average | Average |
| 1 | 0.50 | -n.d.- |
| 5 | 0.45 | 0.77 |
| 10 | 0.44 | 1.05 |

Negative controls showed that the membrane fibers themselves did not substantially influence the results. In the presence and upon binding of chondroitin, the absorbance of DMMB in the measurement range decreases. High absorbance, which leads to negative values, indicates an absence of chondroitin. Generally, polydopamine quantities were found to be in the range of from 0.37 to 0.63 μg/cm² of the membrane surface when coated with a solution as used herein. Chondroitin quantities were in the range of from 0.55 to 1.12 μg/cm².

**Example** 4: **Influence of the PD-CS or PD-CS-HEP coating on the sieving coefficients of membranes**

Various membranes were used for assessing the effect and influence of a polydopamine-chondroitin (PD-CS) and polydopamine-chondroitin-heparin (PD-CS-HEP) coatings according to the invention on membranes. Parameters analyzed included, for example, the sieving coefficients for β2-M, myoglobin, complement factor D, YKL-40 and albumin, as well as the Lp of the respective membranes.

**Example 4.1: Comparison of uncoated minimodules with coated minimodules with 6 mg/mL dopamine and 20 mg(mL chondroitin): sieving coefficients**

Hollow fibers as otherwise used in a commercial hemodialyzer (Theranova 400, Baxter Int., Inc.) were used for preparing a minimodule according to **Example 1.1** and were coated in a one-step approach according to **Example 2.1.** As shown in **Table II,** coating with polydopamine, chondroitin and optionally additionally heparin had a clear influence on the selectivity of the membrane as determined by sieving coefficients for selected marker molecules.

Hollow fibers as otherwise used in commercial hemodialyzers (Theranova, Revaclear, Evodial), or in experimental membranes which have been described before, such as MCO-Ci 400 or MCO 3 (see, for example, Boschetti-de-Fierro et al., Scientific Reports (2015) 5: 18448, wherein MCO 4 corresponds to MCO-Ci 400), all from Baxter, were used for preparing minimodules according to **Example 1.1** and were coated in a one-step approach according to **Example 2.1.**

Another membrane ("TheraX") was prepared and used for coating experiments according to the invention. The TheraX membrane is prepared from a spinning solution containing PAES (13.8%), PVP K85 (1.8%), PVP K30 (4.8%), $H_2O$ (2.6%) and NMP (77.2%) having a viscosity of 3923 cp. Spinning conditions were as described elsewhere (WO 2015/118046 A1, see **Example 1**). The inner diameter of the resulting hollow fibers was 180 μm and the wall thickness was 36 μm.

As shown in the following tables, coating with polydopamine and chondroitin influenced the selectivity performance of the membrane.

***Table II: Influence of differently coating the lumen of hollow fiber membranes from Theranova 400 and 500, MCO 3 and TheraX with polydopamine and chondroitin (Sigma Aldrich) on the sieving coefficient (in %) and the Lp ($\cdot 10^{-4}$cm/bar·s), always in comparison with uncoated samples. Coating and testing were done with minimodules. In each case three samples were tested. Average values determined for each set of samples are provided with standard deviation. "SC Alb" refers to the sieving coefficient of albumin, "SC YKL-40" to the sieving coefficient of YKL-40.***

| # | Membrane | Coating | | Average | | |
|---|---|---|---|---|---|---|
| | | Dopamine [mg/mL] | Chon-droitin [mg/mL] | Lp | SC Alb | SC YKL-40 |
| 1 | Theranova 400 (Lot: 9-6601-H-01) | None | None | 179(±0) | 1.53 (±0.04) | 31.92 (±0.79) |
| 2 | Theranova 400 (Lot 9-6601-H-01) | $H_2O$ | $H_2O$ | 170(±3) | 1.46 (±0.07) | 34.87 (±3.39) |
| 3 | Theranova 400 (Lot 9-6601-H-01) | 6 | - | 160(±8) | 1.82 (±0.22) | 34.70 (±1.24) |
| **4** | **Theranova 400 (Lot 9-6601-H-01)** | **6** | **20** | **128(±4)** | **0.77 (±0.03)** | **31.44 (±1.27)** |
| | | | | | | |
| 5 | Theranova 400 (Lot 9-6603-H-01) | None | None | 157(±6) | 1.05 (±0.02) | 28.30 (±0.78) |
| 6 | Theranova 400 (Lot 9-6601-H-01) | 6 | - | 169(±2) | 1.50 (±0.38 | 35.43 (±2.22 |
| 7 | Theranova 400 (Lot 9-6601-H-01) | - | 20 | 149(±6) | 0.99 (±0.03) | 33.61 (±1.87 |
| **8** | **Theranova 400 (Lot 9-6601-H-01)** | **6** | **20** | **120(±10)** | **0.61 (±0.32)** | **33.62 (±2.69)** |
| | | | | | | |
| 9 | Theranova 500 (Lot: 8-6810-H-01) | None | None | 194(±29) | 1.53 (±0.64) | 37.21 (±6.83) |
| 10 | Theranova 500 (Lot: 8-6810-H-01) | 6 | 20 | 131(±9) | 0.61 (±0.11) | 29.80 (±1.75) |
| 11 | Theranova 500 (Lot: 8-6810-H-01) | 12 | 20 | 105(±5) | 0.23 (±0.02) | 26.37 (±1.60) |
| 12 | Theranova 500 (Lot: 8-6810-H-01) | 6 | 40 | 129(±4) | 0.90 (±0.26) | 32.23 (±2.11) |
| | | | | | | |
| 13 | MCO 3(Nr. 6-804-025-09) | None | None | 193(±5) | 2.46 (±0.51) | 48.19 (±0.99) |
| 14 | MCO 3(Nr. 6-804-025-09) | 6 | - | 212(±9) | 4.06 (±0.78) | 49.66 (±2.87) |
| 15 | MCO 3(Nr. 6-804-025-09) | 6 | 20 | 156(±2) | 1.31 (±0.12) | 45.85 (±3.95) |
| | | | | | | |
| 16 | TheraX (Nr. 001-02) | None | None | 225(±1) | 1.42 (±0.0) | 39.77 (±0.64) |
| 17 | TheraX (Nr. 001-02), stored with residual water (RW) | 6 | 20 | 176(±5) | 1.11 (±0.01) | 44.39 (±0.09) |
| 18 | TheraX (Nr. 001-02) stored after drying with com-pressed air | 6 | 20 | 140(±13) | 0.63 (±0.12) | 31.14 (±2.81) |
| 19 | TheraX (Nr. 006-01, RW) | 6 | 20 | 152(±8) | 1.03 (±0.12) | 47.84 (±0.9) |
| 20 | TheraX (Nr. 006-01, RW, 6 h coat-ing time) | 6 | 20 | 161(±7) | 0.83 (±0.59) | 44.41 (±2.26) |

The results shown in **Table II,** for example for Sample Set 1-4, show that the coating of the base membrane with both dopamine (polydopamine) and chondroitin leads to an improved selectivity of the coated membrane compared to the uncoated membrane. Sample 4, which is coated with polydopamine and chondroitin shows a clear reduction of the sieving coefficient for albumin, whereas the sieving coefficient for YKL-40 remains in the same range. In other words, while the membrane lets pass YKL-40, a representative of the group of larger middle molecules, about as well as the base membrane of the MCO-type (base: 31.92 vs. coated: 31.44), albumin is retained significantly better (base: 1.53 vs. coated: 0.77). This effect is absent when the base membrane is coated with polydopamine only (Sample 3). Sample Set 5-8 corroborates this finding. Again, the combined coating of the base membrane with polydopamine and chondroitin results in an improved selectivity, i.e. a reduced permeability for albumin in conjunction with an increased/maintained permeability for the middle molecule YKL-40. This effect is also absent when the base membrane is coated with a solution only containing chondroitin (Sample 7; base: 1.05 vs. coated: 0.99) In summary, it is evident that the positive influence of the coating on the selectivity of the coated membrane depends on the presence of chondroitin in combination with polydopamine.

Sample Set 9-12 shows the influence of an increase in the concentration of dopamine and chondroitin in the coating solution, respectively. Doubling the concentration of dopamine leads to a further decrease of the albumin sieving coefficient (Sample 11). However, the SC for YKL-40 is also decreased, which is not desirable as the overall selectivity of the membrane does not increase. Doubling the concentration of chondroitin (Sample 12) leads to an increase of the SC for albumin from 0.61 to 0.99%, even though the SC for YKL-40 is increased slightly as well.

Sample Set 13-15 shows that the effect is a general effect and can be obtained also with different membranes. In the said sample set, the MCO 3 membrane was used, which is characterized by a somewhat higher MWRO and MWCO compared to the Theranova 400 membrane. Again, coating the base membrane with polydopamine and chondroitin leads to an increase in the selectivity of the membrane, i.e., the SC for albumin is significantly reduced, whereas the SC for YKL-40 can basically be maintained in the desired range. Sample 14 again shows that the coating with polydopamine only is not having a positive effect on selectivity.

Another membrane, TheraX, was tested in Sample Set 16-20, wherein the emphasis lay mostly on the storing conditions before coating (moist with RW vs. dry, see Samples 17 and 18), and on the influence of the coating time which was increased for Sample 20 vs. 19 from 1h to 6h. It could be shown that the storage conditions of the membranes (minimodules) before coating had no significant influence on the resulting sieving coefficients and selectivity of the coated membranes, and that, accordingly, it is possible to use dry or moist fibers in the process.

The standard coating time used in the experiments performed according to **Example 2** is 1 hour. Increasing the coating (incubation) time seemed to have positive effects in as far as the SC for albumin could be decreased somewhat further, whereas the decrease for the SC of YKL-40 was less pronounced (see Sample Set 19/20 of **Table II**). In terms of industrial applicability shorter coating times will still be preferable. However, also longer coating (incubation) times are feasible, various incubation times can be used depending on the intended outcome.

**Table III** provides for a set of extended data on the influence of a coating according to the invention on the sieving coefficient to β2-M, myoglobin and complement factor D. As can be seen there, the positive effect on sieving coefficients in terms of an increase of selectivity is confirmed for these additional marker molecules.

***Table III:*** *Influence of coating the lumen of hollow fiber membranes from Theranova 400, MCO Ci-400 with polydopamine and chondroitin (Sigma Aldrich) on the sieving coefficient (in %) in comparison with uncoated samples. Coating and testing were done with filters. In each case three samples were tested. "Alb" refers* to *the sieving coefficient of albumin, "YKL-40" to the sieving coefficient of YKL-40, "MYO" refers* to *the sieving coefficient of myoglobin, "β2M" refers* to *the sieving coefficient of β2-M, and "CFD" refers* to *the sieving coefficient of complement factor D.*

| Membrane | Dopa**mine** (mg/mL) | Chondroitin (mg/mL) | Average Sieving Coefficient (%) | | | | |
|---|---|---|---|---|---|---|---|
| | | | B2-M | MYO | HSA | YKL-40 | CFD |
| Theranova 400 (Lot: 9-6605-H-01) | - | - | 95.00 | 80.33 | 0.90 | 35.00 | 50.33 |
| Theranova 400 (Lot: 9-6605-H-01) | 6 | 20 | 97.33 | 68.00 | 0.63 | 37.33 | 50.67 |
| MCO Ci 400 (Lot. 4-500) | - | - | 106.00 | 111.0 0 | 3.50 | 66.33 | 58.00 |
| MCO Ci 400 (Lot. 4-500) | 12 | 20 | 104.67 | 89.33 | 1.03 | 60.33 | 65.00 |

**Example 4.2: Membranes coated with polydopamine, chondroitin and heparin (PD-CS-HEP)**

MCO-Ci 400 membranes were coated in one step with a solution comprising 6 mg/mL dopamine and various amounts of chondroitin and heparin (see **Table IV).** For comparison, MCO-Ci 400 was coated in one step with a solution of 6 mg/mL dopamine and 20 mg/mL chondroitin only.

***Table IV:** Minimodules prepared from MCO Ci-400 membranes and coated with polydopamine, chondroitin and heparin in a one-step procedure versus same membranes coated with polydopamine and heparin. The average Lp as well as the average sieving coefficients (SC) are indicated for albumin (Alb) and YKL-40 based on three measurements, respectively. Comparative samples which were not coated or coated with polydopamine and chondroitin only are shown as well. The chondroitin source is indicated as "SAL" (Sigma Aldrich), "CAR" (Carbosynth") and as "MYT" for the product "Mythocondro®". Sterilization with e-beam was done after drying and with 2g RW.*

| Membrane | Sterilization | Coating | | | **Average** SC (%) | | |
|---|---|---|---|---|---|---|---|
| | | Dopa**mine** (mg/mL) | Chondroitin (mg/mL) | Heparin (mg/mL) | Lp | Alb | YKL-40 |
| MCO Ci 400 (Lot. 4-500-050-03) | no | - | - | - | 277.24 | 2.68 | 46.07 |
| MCO Ci 400 (Lot. 4-500-050-03) | no | 6 | 20 (SAL) | - | 176.24 | 1.13 | 46.35 |
| MCO Ci 400 (Lot. 4-500-050-03) | no | 6 | 19.5 (SAL) | 0.5 | 201.51 | 0.59 | 44.78 |
| MCO Ci 400 (Lot. 0-862) | e-beam (25 kGy) | - | - | - | 283.33 | 2.87 | 51.97 |
| MCO Ci 400 (Lot. 0-862) | e-beam (25kGy) | 3 | 10 (CAR) | 0.1 | 260.22 | 1.83 | 45.41 |
| MCO Ci 400 (Lot. 0-862) | e-beam (25kGy) | 6 | 10 (CAR) | 0.25 | 264.33 | 1.59 | 45.39 |
| MCO Ci 400 (Lot. 0-862) | e-beam (25kGy) | 6 | 19.5 (CAR) | 0.5 | 287.67 | 1.44 | 42.38 |
| MCO Ci 400 (Lot. 0-862) | e-beam (25kGy) | 6 | 5 (CAR) | 0.25 | 269.67 | 1.13 | 43.95 |

Adding heparin to the coating to produce a "triple coating" comprising polydopamine, chondroitin and heparin does not negatively influence the ability of the polydopamine-chondroitin (PD-CS) based coating to increase the selectivity of the treated membrane. However, by adding heparin, it is possible to further introduce an antithrombogenic component which has the potential to further improve the overall clinical performance of the membrane and filter device comprising same (see also **Example** 9) .

**Example 5: Clearance rates achieved with uncoated membranes and membranes coated with PD-CS**

Average clearance rates for uncoated and coated samples (filters) were determined as described in **Example 1.6** in order to better understand the influence of the coating of the invention on the clearance performance of filters. Various membrane types as described in **Example 4.1** were coated as described in **Example 2.1** and clearance rates were determined with the resulting filters. The results are summarized in **Table V.**

***Table V**: Average clearance rates in mL/min for β2-M, myoglobin (MYO), human serum albumin (HSA), YKL-40 and Complement Factor D (CFD) as determined for uncoated and coated filters. Chondroitin used was from Sigma Aldrich. Each value shown is based on three samples/measurements.*

| Membrane | Dopa**mine** (mg/mL) | Chondroitin (mg/mL) | Clearance (mL/min) | | | | |
|---|---|---|---|---|---|---|---|
| | | | B2-M | MYO | HSA | YKL-40 | CFD |
| Theranova 400 (Lot. 9-6606-H-01) | - | - | 108.27 | 67.83 | - | 27.13 | 30.00 |
| Theranova 400 (Lot. 9-6606-H-01) | 6 | 20 | 89.83 | 54.27 | - | 24.30 | 26.17 |
| MCO Ci 400 (Lot. 4-500) | - | - | 115.60 | 90.37 | 1.57 | 38.57 | 33.63 |
| MCO Ci 400 (Lot. 4-500) | 12 | 20 | 111.27 | 83.00 | 1.00 | 38.37 | 36.30 |

**Example 6: Impact of using chondroitin with different molecular weights**

As mentioned in **Example 2,** chondroitin sulfate sodium samples with different average molecular weights (MW) were used for coatings according to the invention in order to assess the influence of MW on the effect of such chondroitin variants on the coating and its impact on the selectivity of a given base membrane. As shown in **Table VI,** all chondroitin variants had an effect on selectivity. The most favorable results were achieved with chondroitin from Carbosynth, i.e., chondroitin with a somewhat higher average molecular weight of between 10 and 50 kDa.

***Table VI**: Influence of chondroitin MW on Lp and selectivity based on sieving coefficients (SC)for albumin (Alb) and YKL-40 ("SAL"=Sigma Aldrich; "MYT"=Mythocondro®; "CAR"=Carbosynth) measured in human plasma and with varied concentrations of dopamine and chondroitin. Three minimodules were tested for each average value provided.*

| Membrane | Dopa**mine** (mg/mL) | Chondroitin (CS) (mg/mL) | CS source | **Average Lp** (·$10^{-4}$ cm/ **(bar ·s)**) | **Average SC (%)** | |
|---|---|---|---|---|---|---|
| | | | | | Alb | YKL-40 |
| *MCO Ci 400 (Lot 4-500-046-09)* | *None* | *None* | *n.a.* | *262.82* | *2.63* | *50.05* |
| MCO Ci 400 (Lot 4-500-046-09) | 12 | 40 | SAL | 177.40 | 1.52 | 50.39 |
| MCO Ci 400 (Lot 4-500-046-09) | 12 | 40 | MYT | 247.98 | 2.08 | 50.00 |
| MCO Ci 400 (Lot 4-500-046-09) | 12 | 40 | CAR | 196.80 | 1.31 | 51.79 |
| MCO Ci 400 (Lot 4-500-046-09) | 6 | 40 | SAL | 189.26 | 2.27 | 52.97 |
| MCO Ci 400 (Lot 4-500-046-09) | 6 | 40 | MYT | 244.89 | 2.46 | 47.97 |
| MCO Ci 400 (Lot 4-500-046-09) | 6 | 40 | CAR | 188.04 | 1.20 | 45.62 |
| MCO Ci 400 (Lot 4-500-047-03) | 12 | 20 | SAL | 181.96 | 1.06 | 47.72 |
| MCO Ci 400 (Lot 4-500-047-03) | 12 | 20 | MYT | 236.58 | 2.44 | 55.24 |
| MCO Ci 400 (Lot 4-500-047-03) | 12 | 20 | CAR | 198.61 | 1.24 | 50.98 |

(continued)

| Membrane | Dopa**mine** (mg/mL) | Chondroitin (CS) (mg/mL) | CS source | **Average Lp (·10$^{-4}$ cm/ (bar ·s))** | **Average SC (%)** | |
|---|---|---|---|---|---|---|
| | | | | | Alb | YKL-40 |
| MCO Ci 400 (Lot 4-500-046-09) | 6 | 20 | SAL* | 288.4 | 2.49 | 47.40 |
| MCO Ci 400 (Lot 4-500-046-09) | 6 | 20 | MYT** | 236.09 | 2.41 | 46.97 |
| MCO Ci 400 (Lot 4-500-046-09) | 6 | 20 | CAR | 211.28 | 1.23 | 46.39 |
| *Data based on only one sample. **Data based on only two samples. | | | | | | |

Example 7: **Coating in recirculation mode**

Coating of minimodules in recirculation mode as described in **Example 2** resulted in equivalent coatings and effects on membrane selectivity, see **Table VII.** Coating is, accordingly, largely independent of the method used and can be adapted to the requirements of the production process for filters.

**Table VII**: *Lp values of membranes and sieving coefficients (SC) for albumin (Alb) and YKL-40 after coating with chondroitin from Carbosynth (CAR, MW=10-50kDa) in recirculation mode without prior sterilization of the tested filters. Measurements were carried out after drying and in the presence of 2g residual water.* The *SC was measured in human plasma. Three minimodules were tested for each average value provided.*

| Membrane | Dopa**mine** (mg/mL) | Chondroitin (CS) (mg/mL) | CS source | Recirculation (min) | Average Lp (**·10$^{-4}$** cm/ (bar · s)) | **Average SC (%)** | |
|---|---|---|---|---|---|---|---|
| | | | | | | Alb | YKL-40 |
| *MCO Ci* 400 *(Lot 0-* | *none* | *none* | *n.a.* | *n.a.* | *237.97* | *2.34* | *48.20* |
| *862)* | | | | | | | |
| *MCO Ci* 400 *(Lot 0-862)* | 6 | 10 | CAR | 30 | 180.00 | 0.30 | 27.89 |
| *MCO Ci* 400 *(Lot 0-862)* | 6 | 10 | CAR | 15 | 194.67 | 0.41 | 35.68 |
| *MCO Ci* 400 *(Lot 0-862)* | 6 | 10 | CAR | 10 | 196.70 | 0.49 | 38.93 |
| *MCO Ci* 400 *(Lot 0-862)* | 6 | 10 | CAR | 5 | 201.57 | 0.69 | 38.33 |

**Example 8: Impact of sterilization on coated filters or minimodules**

As mentioned before, coated filters or minimodules can be sterilized with various methods, such as steam, e-beam or gamma ray sterilization. Preferably, e-beam or gamma ray sterilization is used as it was found that these sterilization methods do not negatively impact the improved filter performance and the integrity of the PD-CS or PD-CS-HEP coating (see **Table VII**), whereas steam sterilization tends to negatively impact the increase in selectivity that is achieved with the coating according to the invention.

***Table VII:*** *Influence of e-beam and gamma sterilization on coatings and selectivity of the coated membranes in minimodules. Sterilization was performed after coating with chondroitin from Carbosynth ("CAR", see also* ***Example 2****) and after drying in the presence of 2g of residual water. Average SCs provided in this Table are based on three measurements.*

| Membrane | Dopamine (mg/mL) | Chondroitin (CS) (mg/mL) | CS source | Sterilization | Average Lp (·$10^{-4}$ cm/(bar ·s)) | Average SC (%) | |
|---|---|---|---|---|---|---|---|
| | | | | | | Alb | YKL-40 |
| *MCO Ci400 (Lot 0-862)* | *none* | *none* | *n.a.* | *e-beam (25 kGy)* | *283.33* | 2.87 | *51.97* |
| *MCO Ci400 (Lot 0-862)* | 6 | 20 | CAR | *e-beam (25 kGy)* | 255.67 | 1.17 | 45.52 |
| *MCO Ci400 (Lot 0-862)* | 12 | 20 | CAR | *e-beam (25 kGy)* | 241.00 | 1.23 | 47.13 |
| *MCO Ci400 (Lot 0-862)* | none | none | n.a. | *gamma (≥ 25kGy)* | 342.33 | 5.10 | 65.13 |
| *MCO Ci400 (Lot 0-862)* | 6 | 20 | CAR | *gamma (≥ 25kGy)* | 258.00 | 1.13 | 48.80 |
| *MCO Ci400 (Lot 0-862)* | 12 | 20 | CAR | *gamma (≥ 25kGy)* | 254.33 | 1.31 | 49.17 |

Filters that were sterilized as described in this Example were also tested for inhibition of cell growth (ICG) as a measure of biocompatibility of the sterilized, coated filters. For determination of ICG, mouse fibroblast cells (L929) are seeded in micro titer plates and cultivated for 24 h in culture medium (250 mL MEM (1x), from Gibco, 25 mL FBS from Gibco, 2.5 mL 200 mM L-glutamine, 2.5 mL 7.5% $NaHCO_3$) until confluency. Cell count before use in the test is done with a Neubauer chamber. Eluate samples from coated and uncoated filters that had been sterilized with either e-beam or gamma rays were prepared in distilled water at 70°C for 24 h. The sample surface area was 10 $cm^2$/mL. Eluates from the tested filters are applied to and incubated with the cells for 72 h at a constant temperature of 37°C after sterile filtration with a 0.2 μm filter. Thereafter, cells were stained with neutral red (25 mL MEM (1x) with 380 μL neutral red solution) for about 180 to 240 minutes. The test relies on the uptake of the dye into the acidic compartments (e.g., lysosomes) of vital cells. According to the amount of bound dye, dye taken up by the cells respectively conclusions can be drawn about the number of living, non-necrotic cells. The dye content is determined photometrically.

***Table VIII:*** *Inhibition of cell growth on uncoated membranes and membranes in minimodules coated according to the invention and sterilized with either e-beam or gamma rays.*

| Membrane | ICG (%) | Treatment |
|---|---|---|
| MCO Ci 400 (Lot 0-862) | 0 | uncoated, gamma |
| MCO Ci 400 (Lot 0-862) | 3 | uncoated, gamma |
| MCO Ci 400 (Lot 4-500-053-09) | 1 | uncoated, gamma |
| MCO Ci 400 (Lot 4-500-053-09) | 2 | uncoated, gamma |
| MCO Ci 400 (Lot 4-500-053-09) | 2 | uncoated, gamma |
| MCO Ci 400 (Lot 0-862) | 6 | uncoated, e-beam |
| MCO Ci 400 (Lot 0-862) | 2 | uncoated, e-beam |
| MCO Ci 400 (Lot 0-862) | 5 | uncoated, e-beam |
| MCO Ci 400 (Lot 0-862) | 3 | uncoated, e-beam |
| MCO Ci 400 (Lot 0-862) | 100 | 12 mg/mL dopamine, gamma |
| MCO Ci 400 (Lot 0-862) | 100 | 12 mg/mL dopamine + 40 mg/mL chondroitin (SAL), gamma |

(continued)

| Membrane | ICG (%) | Treatment |
|---|---|---|
| MCO Ci 400 (Lot 0-862) | 100 | 12 mg/mL dopamine + 40 mg/mL chondroitin (MYT), gamma |
| MCO Ci 400 (Lot 0-862) | 100 | 12 mg/mL dopamine + 40 mg/mL chondroitin (CAR), gamma |
| MCO Ci 400 (Lot 0-862) | 72 | 12 mg/mL dopamine + 40 |
| | | mg/mL chondroitin (SAL), gamma + oxygen scavenger |
| MCO Ci 400 (Lot 0-862) | 8 | 12 mg/mL dopamine + 40 mg/mL chondroitin (SAL), e-beam |
| MCO Ci 400 (Lot 0-862) | 8 | 12 mg/mL dopamine + 40 mg/mL chondroitin (SAL), X-ray |
| MCO Ci 400 (Lot 0-862) | 3 | 12 mg/mL dopamine + 40 mg/mL chondroitin (SAL), e-beam |
| MCO Ci 400 (Lot 0-862) | 6 | 12 mg/mL dopamine + 40 mg/mL chondroitin (SAL), e-beam |
| MCO Ci 400 (Lot 0-862) | 3 | 12 mg/mL dopamine + 40 mg/mL chondroitin (SAL), e-beam |
| MCO Ci 400 (Lot 0-862) | 3 | 12 mg/mL dopamine, e-beam |
| Tube | 10 | untreated reference material |

As shown in **Table VIII,** e-beam sterilization results in a significantly lower ICG compared to gamma sterilized filters (see also **Figure 5**). Without wanting to be bound by theory, it is assumed that gamma sterilization and especially in the presence of oxygen, under the chosen conditions, results in the generation of breakdown or degradation products that negatively affect cell growth. Accordingly, e-beam sterilization seems to be a preferred method for sterilizing PD-CS (or PD-CS-HEP) coated membranes and filters for use in extracorporeal blood treatment applications. The ICG found after e-beam sterilization is well below the threshold of 30% (indicated in **Figure 5** as a dashed line). For a non-toxic material the ICG value must be <30%. Gamma sterilization can still be used if biocompatibility is not a major concern when using, for example, PD-CS coated membranes in other applications. In addition, optimization of gamma sterilization methods may reduce the ICG below the said 30% threshold.

**Example 8: Heparin activity after e-beam sterilization**

As described in **Example 2,** membranes can be coated with a combination of polydopamine, chondroitin and heparin. In addition to increasing selectivity, such coatings have also the ability to reduce the thrombogenicity of the resulting membranes and filters. It is therefore important to maintain the activity of heparin during sterilization of a coated membrane and device.

Accordingly, heparin activity was tested after e-beam sterilization as described above. Heparin activity can be determined, for example, with the heparin anti-Xa assay. The heparin anti-Xa assay is based on the ability of heparin to inhibit the activity of activated factor X (Xa) in the reagent. The reagent includes excess antithrombin, making the heparin in the sample the rate-limiting reagent for Xa inhibition. Heparin in the sample inhibits the enzymatic conversion of a Xa-specific chromogenic substrate to colored product by factor Xa. Standard curves are used to calculate heparin activity in the sample in mU/cm$^2$. The test has been widely used and can be performed according to Newall F., Anti-factor Xa (anti-Xa) assay. Methods Mol Biol. 2013;992:265-72. As can be seen in **Figure 6,** e-beam sterilization of filter modules comprising hollow fiber membranes that have been coated according to the invention with a PD-CS-HEP coating maintains heparin activity.

**Example 9: C3a Activation and hemocompatibility of coated devices according to the invention**

Activation of the complement system by, for example, a medical device that contacts blood, can have deleterious

effects including tissue damage and inflammation. The complement system is comprised of many small proteins and many of them can be used as markers for complement activation. Measurement of, for example, C3a, is suggested by ISO 10993-4.

Minimodules were coated with 12 mg/mL dopamine and 40 mg/mL chondroitin as described in **Example 2.1.** The same minimodules were left uncoated to have a reference. Minimodules comprising Cuprophan (regenerated cellulose) hollow fibers were prepared and used as a positive control. Cuprophan membranes are symmetrically structured and are extremely polar and hydrophilic due to their high proportion of hydroxyl groups. It is known that regenerated cellulose membranes are less biocompatible than modern synthetic membranes such as the MCO Ci 400 membranes used in this experiment. C3a activation was determined with the MicroVue C3a Plus enzyme immunoassay (Quidel, U.S.A.) for determining human C3a concentration in plasma and according to the protocol. The test principle is the quantitative in-vitro determination of the C3a concentration in human plasma, wherein test samples are added to a microtiter plate which is coated with murine monoclonal antibodies which are specific for human C3a. C3a which is available in the test sample is immobilized during incubation. After a washing step, a chromogenic substrate solution (TMB) is added. The bound peroxidase reacts with the substrate and forms a blue color. After stopping the reaction chemically, a blue color appears. The color intensity is proportional to the C3a concentration and can be determined photometrically at 450 nm. **Table IX** shows the results of the C3a determination.

**Table IX:** Concentrations of C3a generated upon incubation of minimodules with human plasma (single pass mode) at 37°C. Samples were taken at 2, 5, 8, 11, 15, 20, 25 and 30 minutes. The concentrations shown are average values based on two (non-coated) and three (coated) minimodules (MCO Ci 400, Lot 4-500-049-11) tested. The regenerated cellulose positive control ("GFE18") was tested only once.

| Time (min) | Concentration C3a (ng/mL) | | |
|---|---|---|---|
| | **Mean MCO-Ci 400 uncoated (n=2) reference** | **Mean MCO-Ci 400 (+12 mg/mL dopamine and 40 mg/mL chondroitin)** | **Regenerated Cellulose GFE18 (pos. control)** |
| 2 | 42,8 | 41,3 | 71,2 |
| 5 | 316,2 | 258,6 | 417,2 |
| 8 | 548,8 | 424,1 | 872,5 |
| 11 | 665,7 | 490,8 | 1299,8 |
| 15 | 899,8 | 562,4 | 1692,9 |
| 20 | 1022,7 | 644,4 | 2510,8 |
| 25 | 1075,9 | 719,9 | 3105,8 |
| 30 | 886,4 | 746,9 | 3213,2 |

**Figure 7** is a graphic representation of the results provided in **Table IX**. It shows that the presence of a coating according to the invention with polydopamine and chondroitin (triangles, dashed line) decreases C3 activation and is, surprisingly, even more hemocompatible than membranes of the state of the art. Accordingly, the coating as described herein provides for an excellent hemocompatibility and supports using a coating according to the invention with hemodialysis membranes and filtration devices for blood treatment.

## Claims

1. A membrane for use in blood treatment comprising a blend of i) a polysulfone, polyethersulfone or polyarylethersulfone and ii) polyvinylpyrrolidone, **characterized in that** a base membrane is coated with polydopamine and chondroitin.

2. A membrane according to claim 1, wherein the membrane is additionally coated with heparin.

3. A membrane according to claim 1 or claim 2, wherein the base membrane is coated with a first layer essentially consisting of polydopamine, a second layer essentially consisting of chondroitin, and optionally additionally a third layer essentially consisting of heparin.

4. A membrane according to claim 1 or claim 2, wherein the base membrane is coated with one layer comprising polydopamine, chondroitin and optionally additionally heparin.

5. A membrane according to claim 2, wherein the base membrane is coated with a first layer comprising polydopamine and chondroitin and a second layer comprising heparin.

6. A membrane according to claim 2, wherein the base membrane is coated with a first layer comprising polydopamine and a second layer comprising chondroitin and heparin.

7. A membrane according to any of claims 1 to 6, wherein the base membrane is coated with polydopamine in an amount of from 0.30 $\mu g/cm^2$ to 1.10 $\mu g/cm^2$ of the membrane surface.

8. A membrane according to any of claims 1 to 7, wherein the base membrane is coated with chondroitin in an amount of from 0.30 $\mu g/cm^2$ to 1.20 $\mu g/cm^2$ of the membrane area.

9. A membrane according to any of claims 2 to 8, wherein the base membrane is coated with heparin in an amount of from 5 $mU/cm^2$ to 100 $mU/cm^2$ of the membrane surface.

10. A membrane according to any of claims 1 to 9 wherein the base membrane is a MCO membrane having a MWRO of from 8.5 to 14.0 kDa and a MWCO of from 50.0 to 130.0 kDa as measured by dextran sieving before blood contact.

11. A membrane according to any of claims 1 to 10, wherein the base membrane is an HCO membrane having a MWRO of from 15kDa to 20kDa and a MWCO of from 170kDa to 320kDa as measured by dextran sieving before blood contact.

12. A membrane according to any of claims 1 to 11, wherein the membrane is a flat sheet membrane or a hollow fiber membrane.

13. A method of increasing the selectivity of a flat sheet or hollow fiber membrane comprising the step of coating the membrane with polydopamine and chondroitin and optionally additionally with heparin.

14. A process for preparing a membrane according to claim 1 or claim 2, comprising the steps of

(a) Providing a membrane comprising a blend of i) a polysulfone, polyethersulfone or polyarylethersulfone and ii) polyvinylpyrrolidone;
(b) Providing a dopamine solution comprising dopamine in an amount of from 2 mg/mL to 80 mg/mL at a pH of from 8.0 to 10.0;
(c) Dissolving chondroitin in an amount of from 5 mg/mL and 80 mg/mL in the solution of step (b);
(d) Optionally adding heparin to the solution of step (b) or (c) in an amount of from 0.1 mg/mL to 100 mg/mL;
(e) Contacting the membrane surface with the solution of step (c) or step (d);
(f) Rinsing and optionally drying the coated membrane;
(g) Optionally sterilizing the coated membrane.

15. A process for preparing a membrane according to claim 14, wherein step (b) is followed by the step of

(c) Contacting the membrane surface with the solution of step (c) or step (d);
(d) Rinsing and optionally drying the coated membrane;
(e) Providing a solution of chondroitin in an amount of from 5 mg/mL and 80 mg/mL;
(f) Optionally adding heparin to the solution of step (e) in an amount of from 0.1 mg/mL to 100 mg/mL;
(g) Contacting the membrane surface with the solution of step (e) or (f);
(h) Rinsing and optionally drying the membrane of step (g);
(i) Optionally sterilizing the coated membrane.

16. A process for preparing a membrane according to claim 15, wherein step (e) is followed by the step of

(f) Contacting the membrane surface with the solution of step (e);
(g) Rinsing and optionally drying the membrane of step (f);
(h) Providing a solution of heparin in an amount of from 0.1 mg/mL to 100 mg/mL;
(i) Contacting the membrane surface with the solution of step (h);

(j) Rinsing and optionally drying the membrane of step (i);
(k) Optionally sterilizing the coated membrane.

**17.** A dialyzer for blood treatment comprising a membrane according to any of claims 1 to 12.

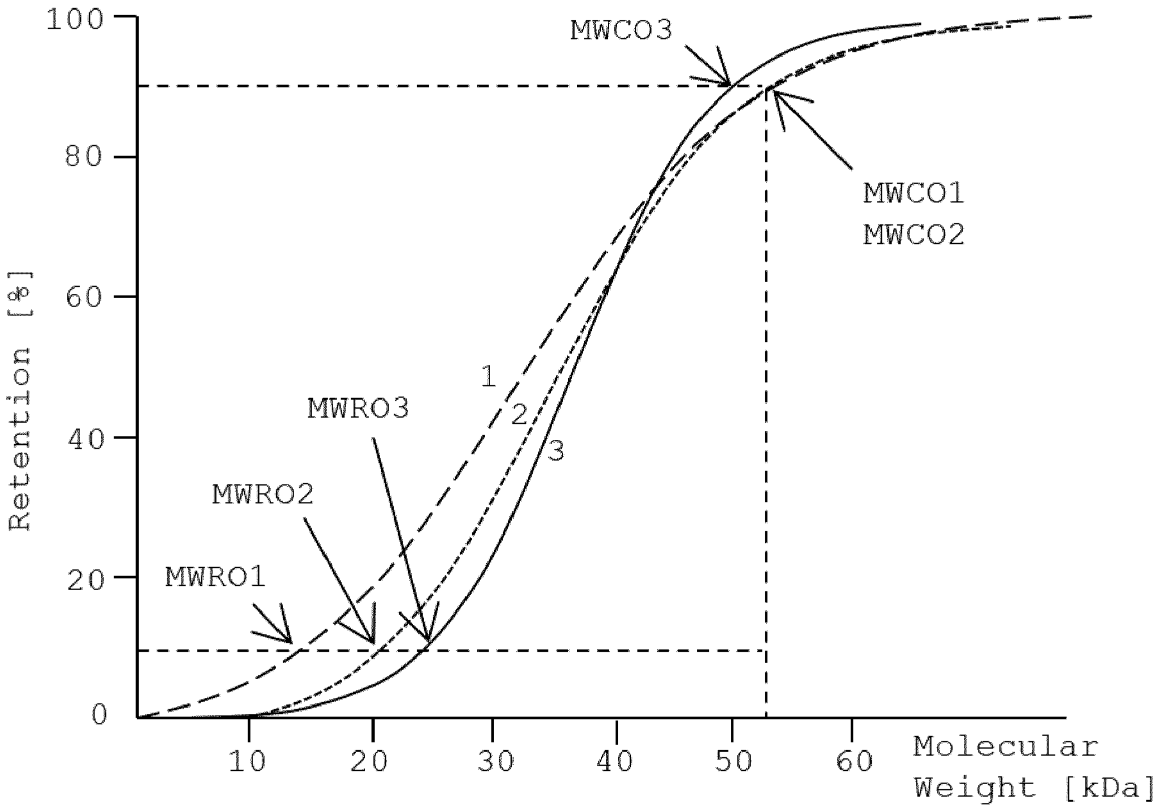
100
80
60
40
20
0
Retention [%]
10
20
30
40
50
60
Molecular
Weight [kDa]
MWCO3
MWCO1
MWCO2
MWRO3
MWRO2
MWRO1
1
2
3

Figure 1

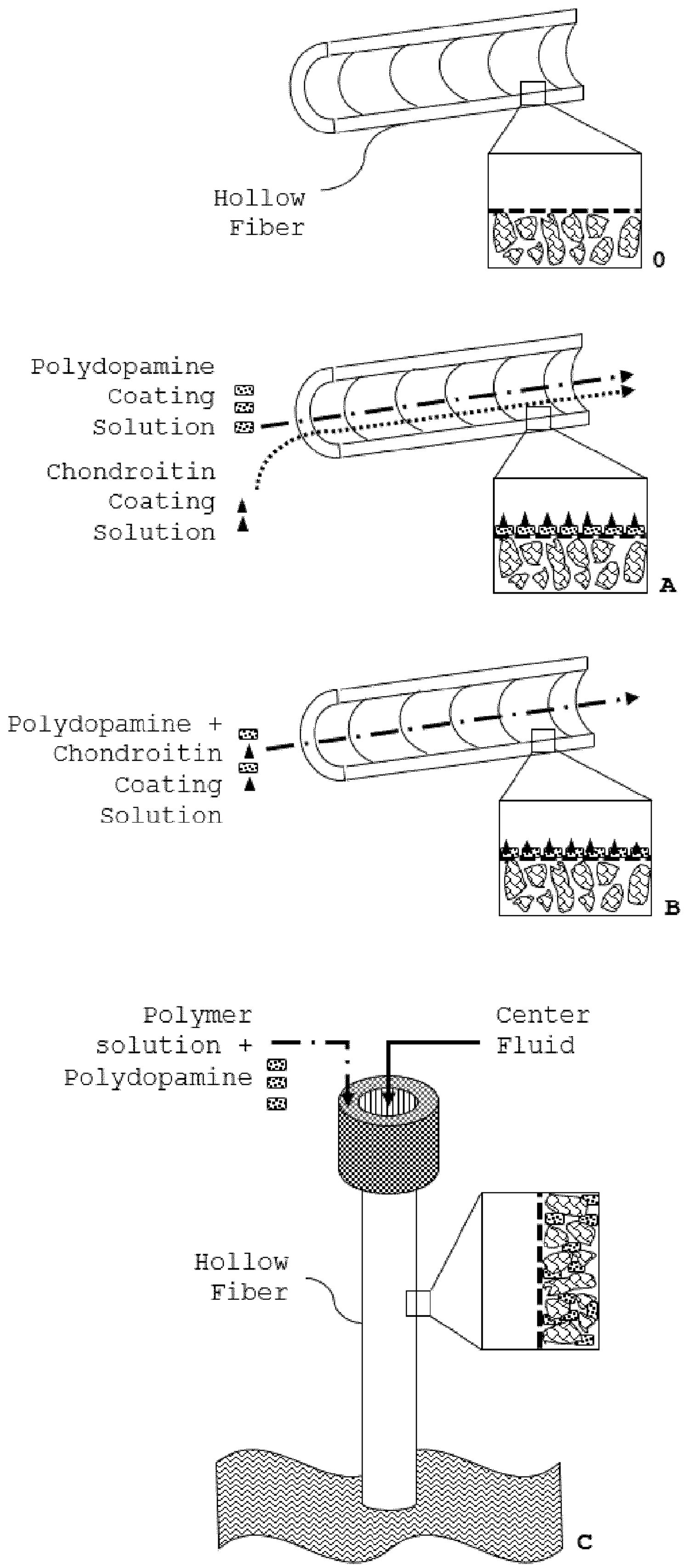
Hollow
Fiber
0
Polydopamine
Coating
Solution
Chondroitin
Coating
Solution
A
Polydopamine +
Chondroitin
Coating
Solution
B
Polymer
solution +
Polydopamine
Center
Fluid
Hollow
Fiber
C

Figure 2

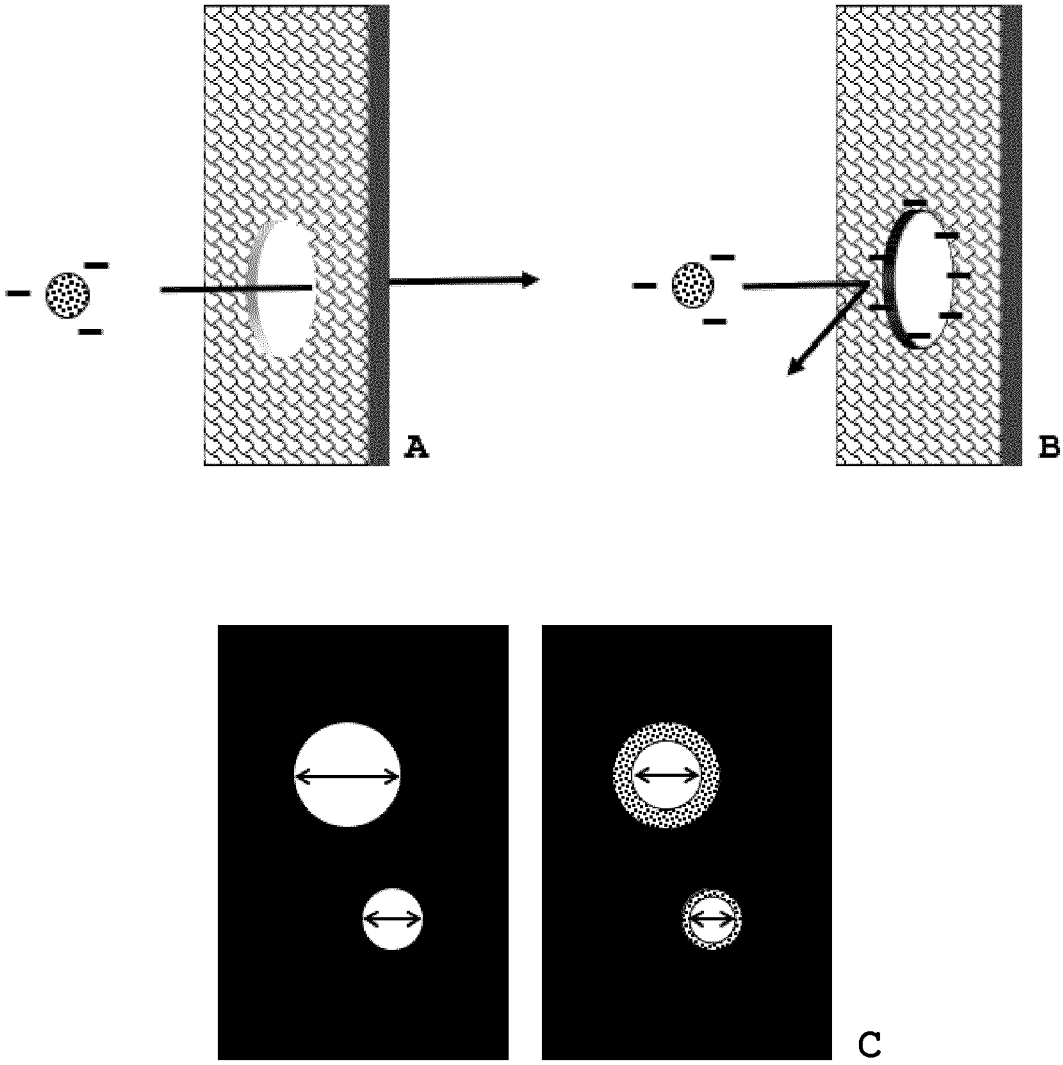


Figure 3

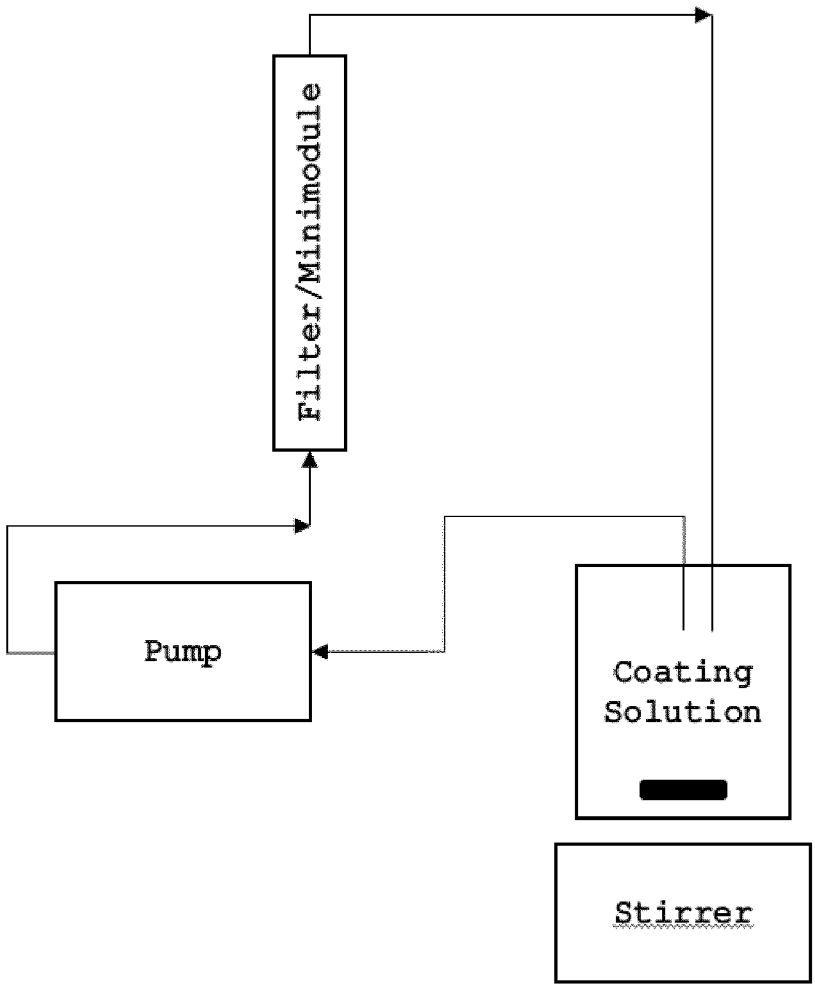


Figure 4

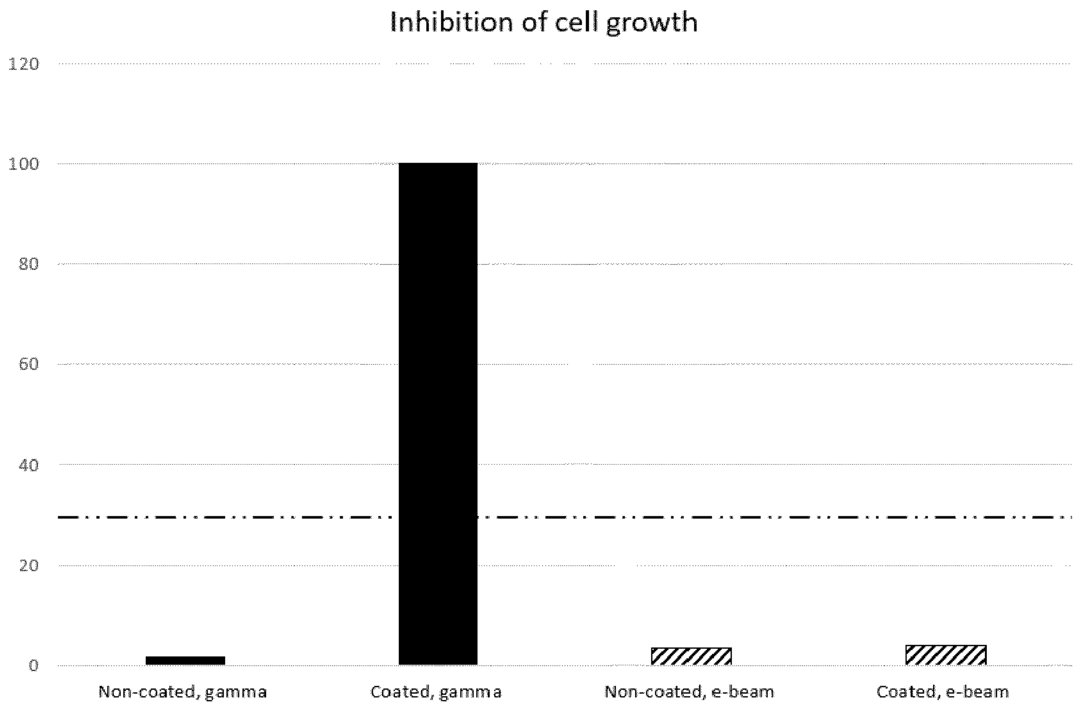
Inhibition of cell growth
120
100
80
60
40
20
0
Non-coated, gamma
Coated, gamma
Non-coated, e-beam
Coated, e-beam

Figure 5

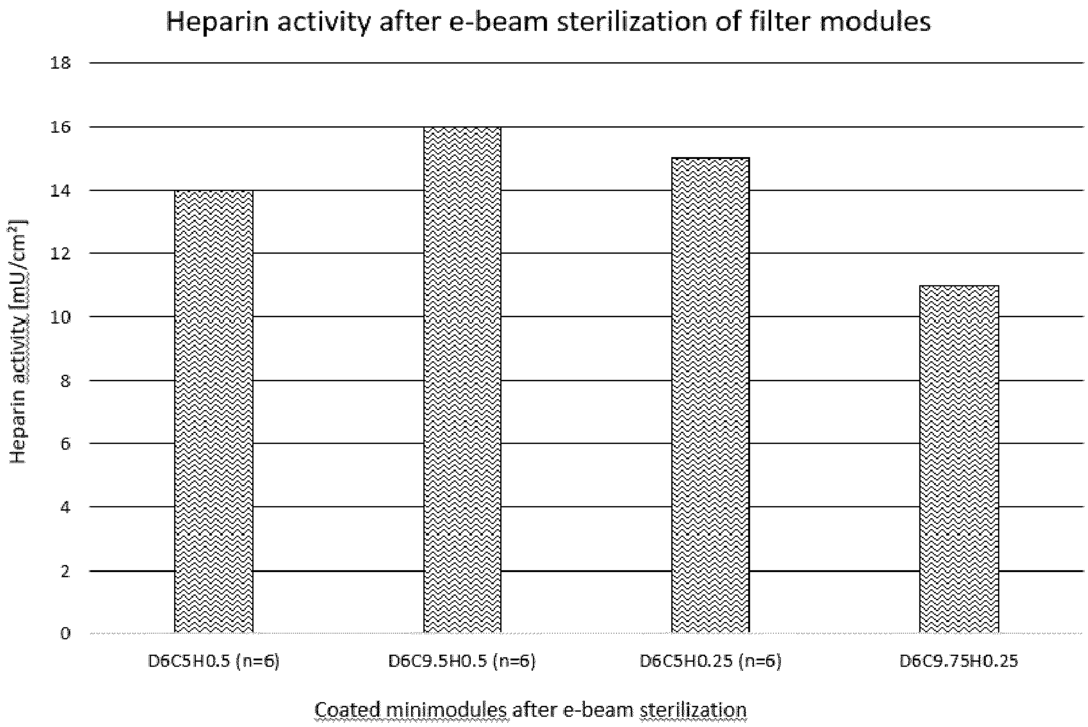
Heparin activity after e-beam sterilization of filter modules
18
16
14
12
10
8
6
4
2
0
Heparin activity [mU/cm²]
D6C5H0.5 (n=6)
D6C9.5H0.5 (n=6)
D6C5H0.25 (n=6)
D6C9.75H0.25
Coated minimodules after e-beam sterilization

Figure 6

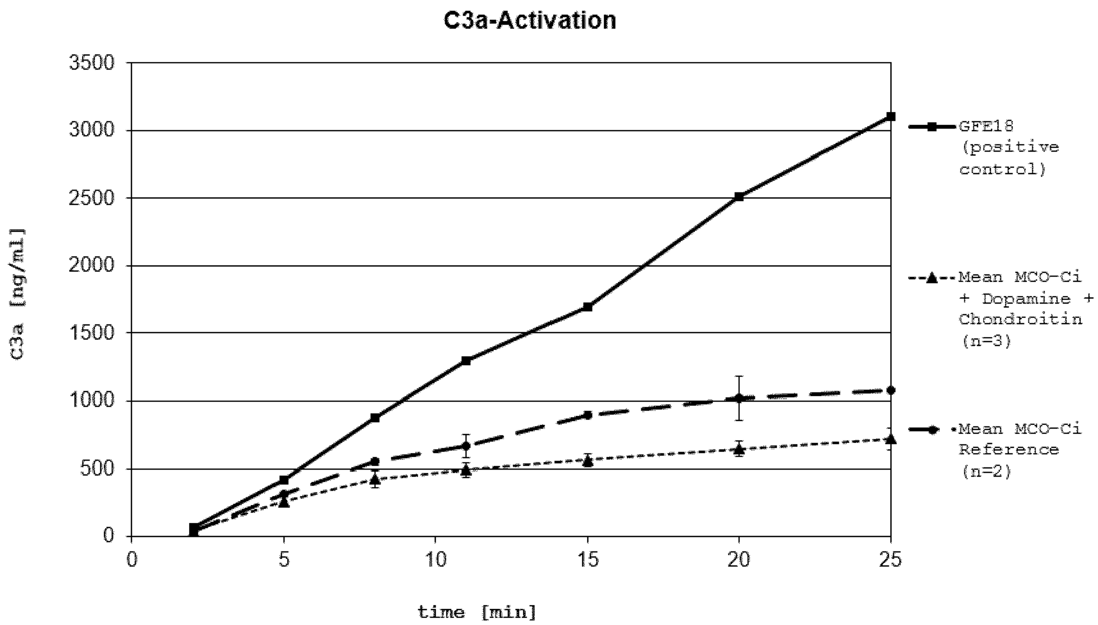
C3a-Activation
3500
3000
2500
2000
1500
1000
500
0
C3a [ng/ml]
0
5
10
15
20
25
time [min]
GFE18 (positive control)
Mean MCO-Ci + Dopamine + Chondroitin (n=3)
Mean MCO-Ci Reference (n=2)

Figure 7

Europäisches Patentamt
European Patent Office
Office européen des brevets

# EUROPEAN SEARCH REPORT

Application Number
EP 21 21 6683

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | REN XIAOZHI ET AL: "Aligned porous fibrous membrane with a biomimetic surface to accelerate cartilage regeneration", CHEMICAL ENGENEERING JOURNAL, vol. 370, 1 August 2019 (2019-08-01), pages 1027-1038, XP55926868, AMSTERDAM, NL ISSN: 1385-8947, DOI: 10.1016/j.cej.2019.03.271 | 13 | INV. B01D67/00 A61M1/34 B01D71/44 B01D71/68 |
| Y | * abstract * * paragraphs [02.2] - [02.3] * ----- | 1-12, 14-17 | |
| Y | XIE BINGWU ET AL: "A Decoration of heparin and bovine serum albumin on polysulfone membrane assisted via polydopamine strategy for hemodialysis", JOURNAL OF BIOMATERIALS SCIENCE. POLYMER EDITION, VSP, UTRECHT, NL , vol. 27, no. 9 12 June 2016 (2016-06-12), pages 880-897, XP009535996, ISSN: 0920-5063, DOI: 10.1080/09205063.2016.1169479 Retrieved from the Internet: URL:http://www.tandfonline.com/doi/full/10.1080/09205063.2016.1169479 [retrieved on 2016-04-22] * abstract * * paragraphs [02.1] - [02.2] * ----- | 1-17 | TECHNICAL FIELDS SEARCHED (IPC) B01D A61M |
| Y | WO 2015/118046 A1 (GAMBRO LUNDIA AB [SE]) 13 August 2015 (2015-08-13) * claims * ----- -/-- | 10,11 | |

The present search report has been drawn up for all claims

1

| Place of search | Date of completion of the search | Examiner |
|---|---|---|
| Munich | 2 June 2022 | Veríssimo, Sónia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 21 21 6683

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YUAN N Y ET AL: "Fabrication and characterization of chondroitin sulfate-modified chitosan membranes for biomedical applications", DESALINATION, ELSEVIER, AMSTERDAM, NL, vol. 234, no. 1-3, 31 December 2008 (2008-12-31), pages 166-174, XP025712397, ISSN: 0011-9164, DOI: 10.1016/J.DESAL.2007.09.083 [retrieved on 2008-11-05] * abstract * ----- | 1-17 | |
| Y | HAN LU ET AL: "Mussel-Inspired Tissue-Adhesive Hydrogel Based on the Polydopamine-Chondroitin Sulfate Complex for Growth-Factor-Free Cartilage Regeneration", APPLIED MATERIALS & INTERFACES, vol. 10, no. 33, 22 August 2018 (2018-08-22), pages 28015-28026, XP55926884, US ISSN: 1944-8244, DOI: 10.1021/acsami.8b05314 * abstract * ----- | 1-17 | TECHNICAL FIELDS SEARCHED (IPC) |
| A | CN 103 480 278 A (YANTAI LVSHUIFU MEMBRANE MATERIAL LTD) 1 January 2014 (2014-01-01) * claims * ----- -/-- | 1-17 | |

The present search report has been drawn up for all claims

1

| Place of search | Date of completion of the search | Examiner |
|---|---|---|
| Munich | 2 June 2022 | Veríssimo, Sónia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 21 21 6683

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZHANG BO ET AL: "Improved Antithrombogenicity of a Poly(lactic acid) Surface Grafted with Chondroitin Sulfate", ACS APPLIED BIO MATERIALS, vol. 4, no. 3, 15 March 2021 (2021-03-15), pages 2696-2703, XP55927315, US ISSN: 2576-6422, DOI: 10.1021/acsabm.0c01629 * abstract * * paragraph [0001] * ----- | 1-17 | |
| | | | TECHNICAL FIELDS SEARCHED (IPC) |

The present search report has been drawn up for all claims

| Place of search | Date of completion of the search | Examiner |
|---|---|---|
| Munich | 2 June 2022 | Veríssimo, Sónia |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 21 6683

This annex lists the patent family members relating to the patent documents cited in the above-mentioned European search report. The members are as contained in the European Patent Office EDP file on
The European Patent Office is in no way liable for these particulars which are merely given for the purpose of information.

02-06-2022

| Patent document cited in search report | Publication date | Patent family member(s) | Publication date |
|---|---|---|---|
| WO 2015118046 A1 | 13-08-2015 | AU 2015214950 A1 | 04-08-2016 |
| | | AU 2018278913 A1 | 03-01-2019 |
| | | CA 2938222 A1 | 13-08-2015 |
| | | CN 105722582 A | 29-06-2016 |
| | | CN 111545068 A | 18-08-2020 |
| | | EP 3102312 A1 | 14-12-2016 |
| | | EP 3427814 A1 | 16-01-2019 |
| | | ES 2701847 T3 | 26-02-2019 |
| | | HK 1224246 A1 | 18-08-2017 |
| | | JP 6636437 B2 | 29-01-2020 |
| | | JP 6924253 B2 | 25-08-2021 |
| | | JP 2017507706 A | 23-03-2017 |
| | | JP 2020039952 A | 19-03-2020 |
| | | KR 20160119190 A | 12-10-2016 |
| | | KR 20210129258 A | 27-10-2021 |
| | | PL 3102312 T3 | 31-05-2019 |
| | | PT 3102312 T | 18-12-2018 |
| | | US 2017165615 A1 | 15-06-2017 |
| | | US 2020316533 A1 | 08-10-2020 |
| | | US 2022152563 A1 | 19-05-2022 |
| | | WO 2015118046 A1 | 13-08-2015 |
| CN 103480278 A | 01-01-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015118046 A1
- US 20030021826 A1
- WO 2012047282 A2
- US 20100059433 A1
- WO 2013016574 A1
- US 20160101390 A1
- EP 2695668 A1
- WO 2017015291 A1
- WO 2017030502 A1
- WO 2015118045 A1
- WO 2004056460 A1


### Non-patent literature cited in the description


- **BOSCHETTI-DE-FIERRO et al.** *Scientific Reports,* 2015, vol. 5, 18448
- **ROSNER et al.** *Classification of Uremic Toxins and Their Role in Kidney Failure: CJASN,* 2021, vol. 16, 1-8
- **LORENZ et al.** *Kidney International,* 2018, vol. 93, 221-230
- **KIRSCH et al.** *Nephrology Dialysis Transplantation,* 2017, vol. 32, 165
- **LIEBSCHER.** Chemistry of Polydopamine - Scope, Variation, and Limitation. *Eur. J. Org. Chem.,* 2019, vol. 2019, 4976-4994
- **GAO et al.** *Journal of Membrane Science,* 2014, vol. 452, 390-399
- **LI et al.** *Desalination,* 2014, vol. 344, 422-430
- **VOLPI.** *molecules,* 2019, vol. 24, 1447
- **NING et al.** *Progress in Polymer Science,* 2018, vol. 81, 144-162
- **HAN et al.** *ACS Applied Materials & Interfaces,* 2018, vol. 10, 28015-28026
- **YUAN et al.** *Desalination,* 2008, vol. 234, 166
- **REN et al.** *Chemical Engineering Journal,* 2019, vol. 370, 1027-1038
- **HUANG et al.** *Acta Biomaterialia,* 2018, vol. 71, 184-199
- **RONCO ; CLARK.** Nature Reviews. *Nephrology,* 2018, vol. 14 (6), 394-410
- **GONDOUIN ; HUTCHISON.** High Cut-Off Dialysis Membranes: Current Uses and Future Potential. *Advances in Chronic Kidney Disease,* 2011, vol. 18, 180-187
- **ROSNER et al.** Classification of Uremic Toxins and Their Role in Kidney Failure. *Clin J Am Soc Nephrol,* 2021
- **SALOMÄKI et al.** *J. Phys. Chem. B,* 2018, vol. 122, 6314-6327
- **LANER-PLAMBERGER et al.** *Int. J. Mol. Sci.,* 2021, vol. 22, 12041
- **SAID et al.** A Review of Commercial Developments and Recent Laboratory Research of Dialyzers and Membranes for Hemodialysis Application. *Membranes,* 2021, vol. 11, 767
- **SMITH et al.** *Analytical Biochemistry,* 1985, vol. 150, 76-85
- **FARNDALE et al.** *Biochimica et Biophysica Acta,* 1986, vol. 883, 173-177
- **FIERRO et al.** *Scientific Reports,* 2015, vol. 5, 18448
- **NEWALL F.** Anti-factor Xa (anti-Xa) assay. *Methods Mol Biol,* 2013, vol. 992, 265-72